# EUROPEAN PATENT APPLICATION

(11) **EP 4 609 840 A1**
(43) Date of publication of application: **03.09.2025**
(21) Application number: 25160525.9
(22) Date of filing: 27.02.2025
(51) Int. Cl.: A61F 9/008

(54) **OPHTHALMIC LASER TREATMENT DEVICE**

(30) Priority: 28.02.2024 JP 2024029101; 28.02.2024 JP 2024029102
(71) Applicant: NIDEK CO., LTD., Gamagori-shi, Aichi 443-0038 (JP)
(72) Inventor: NAKAI, Shigeyoshi, Gamagori-shi, Aichi, 443-0038 (JP); MASUNAGA, Kohei, Gamagori-shi, Aichi, 443-0038 (JP); TSUKAMOTO, Teruki, Gamagori-shi, Aichi, 443-0038 (JP)
(74) Representative: Viering, Jentschura & Partner mbB Patent- und Rechtsanwälte

(57) **Abstract**

An ophthalmic laser treatment device includes a control unit that executes a guide display step of controlling a display unit to display a guide in accordance with a progress of an irradiation plan. The guide is used to align a target position on a tissue of a patient's eye with a next irradiation spot of a plurality of irradiation spots at which treatment laser light is scheduled to be emitted next time. The control unit further executes an irradiation position movement step of moving an irradiation position of the treatment laser light and an aiming light by controlling an irradiation position moving unit.

## Description

### TECHNICAL FIELD

The present disclosure relates to an ophthalmic laser treatment device that treats tissue of a patient's eye by irradiating the tissue with treatment laser light.

### BACKGROUND

Techniques have been proposed to assist in treating patient's eyes using treatment laser light. For example, an ophthalmic laser treatment device disclosed in JP 2014-233469 A displays a target area to be irradiated with the treatment laser light on a display screen, and also displays an area that is actually irradiated with the treatment laser light (a post-irradiation area) when the treatment laser light is irradiated. The target irradiation area and the irradiated area are displayed for the purpose of allowing the operator to check the progress of the treatment.

### SUMMARY

For treating a subject eye's tissue with a treatment laser light, a contact lens having a reflective surface to reflect the treatment laser light is sometimes used. For example, a contact lens having a reflective surface is sometimes used to treat a ring-shaped trabecular meshwork that exists in the area where the cornea and iris of the eyeball are in contact with each other. In this case, the operator needs to adjust the aiming point for the treatment laser light by observing the tissue reflected on the reflective surface of the contact lens while holding the contact lens. Furthermore, when irradiating multiple portions of the tissue with treatment laser light, the operator needs to adjust the aiming point of the treatment laser light while rotating the reflective surface of the contact lens appropriately. Thus, skill of the operator is required to accurately adjust the aiming point. In view of the above, there has been a demand for a technique that can appropriately support treatment with a treatment laser light when a contact lens having a reflective surface is used.

A typical objective of the present disclosure is to provide an ophthalmic laser treatment device that is configured to appropriately support treatment with a treatment laser light when a contact lens having a reflective surface is used.

In one aspect of the present disclosure, an ophthalmic laser treatment device irradiates a tissue of a patient's eye with treatment laser light in response to receiving an irradiation execution instruction. The ophthalmic laser treatment device includes: a treatment laser light source that is configured to emit treatment laser light; an aiming light source that is configured to emit aiming light for an operator to recognize a scheduled irradiation position for the treatment laser light on the tissue; an irradiation position moving unit that is configured to move, on the tissue, an irradiation position at which the treatment laser light and the aiming light are emitted; an observation optical system that is configured to present an observation image of the tissue to the operator; a display unit that is configured to display an image by superimposing the image on the observation image observed by the operator; and a control unit. The control unit is configured to execute: an irradiation plan acquisition step of acquiring an irradiation plan that defines an irradiation order of irradiations with the treatment laser light to a plurality of irradiation spots at which the treatment laser light is scheduled to be emitted when irradiating the patient's eye with the treatment laser light using a contact lens having a reflective surface that reflects the treatment laser light and the aiming light in a direction intersecting an optical axis; a guide display step of controlling the display unit to display a guide in accordance with a progress of the irradiation plan, wherein the guide is used to align a target position on the tissue with a next irradiation spot of the plurality of irradiation spots at which the treatment laser light is scheduled to be emitted next time; and an irradiation position movement step of moving the irradiation position of the treatment laser light and the aiming light by controlling the irradiation position moving unit.

In a second aspect of the present disclosure, an ophthalmic laser treatment device irradiates a tissue of a patient's eye with treatment laser light in response to receiving an irradiation execution instruction. The ophthalmic laser treatment device includes: a treatment laser light source that is configured to emit treatment laser light; an aiming light source that is configured to emit aiming light for an operator to recognize a scheduled irradiation position for the treatment laser light on the tissue; an irradiation position moving unit that is configured to move, on the tissue, an irradiation position at which the treatment laser light and the aiming light are emitted; an observation optical system that is configured to present an observation image of the tissue to the operator; a display unit that is configured to display an image by superimposing the image on the observation image observed by the operator; and a control unit. The control unit is configured to execute: an irradiation plan acquisition step of acquiring an irradiation plan that defines an irradiation order of irradiations with the treatment laser light to a plurality of irradiation spots at which the treatment laser light is scheduled to be emitted when irradiating the patient's eye with the treatment laser light using a contact lens having a reflective surface that reflects the treatment laser light and the aiming light in a direction intersecting an optical axis; a guide display step of controlling the display unit to display a guide in accordance with a progress of the irradiation plan, wherein the guide is used to align a target position on the tissue with a next irradiation spot of the plurality of irradiation spots at which the treatment laser light is scheduled to be emitted next time. At the guide display step, the control unit is further configured to execute: when irradiation with the treatment laser light to n irradiation spots of the plurality of irradiation spots is completed, a non-adjacent position change step of changing a next aiming point, which is used to set the target position on the tissue, to a non-adjacent position, wherein the non-adjacent position is different from an adjacent position that is offset from a previous irradiation spot of the plurality of irradiation spots by n intervals of the plurality of irradiation spots in a forward direction defined by the irradiation plan; and when executing the non-adjacent position change step, a target position indicator display step of controlling the display unit to display a target position indicator, wherein the target position indicator indicates the tissue at the adjacent position as the target position with which the next aiming point of the guide is aligned.

According to the ophthalmic laser treatment device in the present disclosure, it is possible to appropriately support treatment with a treatment laser light when a contact lens having a reflective surface is used.

### BRIEF DESCRIPTION OF DRAWINGS

FIG. 1 is an external view of an ophthalmological laser treatment device.
FIG. 2 is a side view of an optical system of the ophthalmic laser treatment device.
FIG. 3 is a diagram of the optical system of the ophthalmic laser treatment device when viewed from an upper side.
FIG. 4 is a schematic cross-sectional view of a patient's eye and a contact lens.
FIG. 5 is a diagram schematically showing an example of an observation site observed through a reflective surface of the contact lens.
FIG. 6 is an exploded perspective view of the contact lens that is a partially rotated lens.
FIG. 7 is a view of the contact lens provided with an interval indicator when viewed from a side opposite to the side that is in contact with the patient's eye.
FIG. 8 is a diagram showing an example of an irradiation plan displayed on a control box.
FIG. 9 is an explanatory diagram for explaining a method of adjusting an aiming point of an irradiation spot.
FIG. 10 is a diagram showing an example of an operator's observation field of view during treatment by the ophthalmic laser treatment device according to a first embodiment.
FIG. 11 is a diagram showing an example of transition of a part of the operator's observation field during treatment by the ophthalmic laser treatment device according to the first embodiment.
FIG. 12 is a flowchart of treatment control processing executed by the ophthalmic laser treatment device according to the first embodiment.
FIG. 13 is a diagram showing an example of an operator's observation field of view during treatment by an ophthalmic laser treatment device according to a second embodiment.

### DETAILED DESCRIPTION

### <Overview>

In a first aspect of the present disclosure, an ophthalmic laser treatment device irradiates a tissue of a patient's eye with treatment laser light in response to receiving an irradiation execution instruction. The ophthalmic laser treatment device includes a treatment laser light source, an aiming light source, an irradiation position moving unit, an observation optical system, a display unit, and a control unit. The treatment laser light source is configured to emit treatment laser light. The aiming light source is configured to emit aiming light for an operator to recognize a scheduled irradiation position for the treatment laser light on the tissue. The irradiation position moving unit is configured to move, on the tissue, an irradiation position at which the treatment laser light and the aiming light are emitted. The observation optical system is configured to present an observation image of the tissue to the operator. The display unit is configured to display an image by superimposing the image on the observation image observed by the operator. The control unit executes an irradiation plan acquisition step, a guide display step, and an irradiation position movement step. In the irradiation plan acquisition step, when irradiating the patient's eye with the treatment laser beam using a contact lens having a reflective surface that reflects the treatment laser light and the aiming light in a direction intersecting the optical axis, the control unit acquires an irradiation plan in which the order of irradiation of treatment laser light is set for a plurality of irradiation spots to be irradiated. At the guide display step, the control unit displays a guide on the display unit in accordance with a progress of the irradiation plan, wherein the guide is displayed to align a target position on the tissue with a next one of the plurality of irradiation spots at which the treatment laser light is scheduled to be emitted next time. At the irradiation position movement step, the control unit moves the irradiation position of the treatment laser light and the aiming light by controlling the irradiation position moving unit.

According to the ophthalmic laser treatment device in the present disclosure, the display unit displays a guide according to the progress of an irradiation plan. The guide is displayed to make a target position on the tissue at which treatment laser light is to be emitted match at least a next irradiation spot at which the treatment laser light will be emitted next time. Thus, an operator can visually recognize the guide while observing the observation image of the tissue of the patient's eye and adjust the position of the irradiation spots for the treatment laser light on the tissue by referring to the recognized guide. Thus, the operator can easily and appropriately irradiate each of the plurality of irradiation spots. Further, in the ophthalmic laser treatment device according to the present disclosure, when the relative position of the device relative to the patient's eye (hereinafter simply referred to as the "relative position of the device") moves, the position of the optical axes of the treatment laser light and the aiming light also move. Thus, the irradiation position of the treatment laser light and the aiming light on the tissue move. Further, when the contact lens is rotated, the angle of the reflective surface of the contact lens changes, and thus the aiming point of the treatment laser light and the aiming light on the tissue moves. However, the amount of movement of the observation image observed by the operator increases as the amount of movement in the relative position of the device and the amount of rotation of the contact lens increases. Thus, it would be difficult to adjust the aiming point to an appropriate position on the tissue. Furthermore, the efficiency of treatment work would also decrease as the frequency of changing the relative position of the device and the angle of the contact lens increases. In contrast, according to the ophthalmic laser treatment device in the present disclosure, the irradiation position moving unit moves the irradiation position of treatment laser light and the target light on the tissue of the patient's eye. Thus, the operator can easily adjust the arrangement of the irradiation spot on the tissue while the amount of movement of the observed image is reduced. The amount of operation performed by the operator can be also reduced. Accordingly, it is possible to appropriately assist the operator in treatment with treatment laser light when a contact lens having a reflective surface is used.

According to the techniques exemplified in the present disclosure, for example, selective laser trabeculonoplasty (SLT) and micropulse laser trabeculoplasty (MICROPULSE LASER TRABECULATY: MLT), etc., are appropriately supported. SLT is a treatment that lowers eye pressure in a patient's eye by irradiating the trabecular meshwork with a laser and destroying pigment cells in the trabecular meshwork. MLT is a treatment that lowers eye pressure in a patient's eye by irradiating the trabecular meshwork with laser light having lower energy than lasers used in SLT and activating trabecular meshwork cells to improve aqueous humor flow. Note, MLT is less likely to leave irradiation marks by the treatment laser light. When no irradiation marks from treatment laser light remain, the operator cannot recognize the previous irradiation position of the treatment laser light from the irradiation marks. Thus, it is difficult to accurately set the irradiation spots on the tissue. On the other hand, according to the technology exemplified in the present disclosure, treatment with treatment laser light is appropriately supported by the guide, etc., even when no irradiation marks remain. Therefore, the accuracy and efficiency of treatment can be easily improved.

The configuration of the observation optical system for the operator to observe an observation image of the patient's eye tissue can be selected as appropriate. For example, the observation optical system allows an operator to observe an observed image of the patient's eye through an eyepiece. Also, the observation optical system may allow an operator to observe the observation image of the patient's eye by capturing an image of the tissue of the patient's eye and displaying the captured image on a display device (for example, a monitor or projector, etc.).

Furthermore, the configuration of the display unit for superimposing and displaying images on the observation image observed by the operator can also be appropriately selected. For example, when an operator observes an observation image via an eyepiece, the display unit may superimpose the image on the observation image observed via the eyepiece. In this case, the display unit may be configured to synthesize the optical observation image observed by the operator and the image displayed on a display device and project the synthesized image onto the operator's eye. Further, the display unit may be configured to project an image onto the retina of the operator's eye by scanning the retina with light rays. In other words, the display unit may be a so-called head-up display (HUD). Furthermore, when a display device (monitor, etc.) displays the observation image to an operator by displaying the captured image (the observation image), the display device may superimpose an image such as a spot interval guide on the observation image. In other words, a display device may commonly serve to provide a partial function of the observation optical system and a partial function of the display unit.

The configuration of the irradiation position moving unit for moving the irradiation position of the treatment laser light and the aiming light can also be appropriately selected. The irradiation position moving unit moves the irradiation position of the treatment laser light and the aiming light by changing the position of the optical axes of the treatment laser light and the aiming light with respect to the optical axis of the observation optical system. In other words, the irradiation position moving unit may move the irradiation position of the treatment laser light and the aiming light while maintaining the position of the observation optical axis of the observation optical system. For example, the irradiation position moving unit may include a deflection unit (for example, a galvanometer mirror, etc.) that changes the direction of deflection of the treatment laser light and the aiming light. Further, an irradiation position moving unit may move an optical member (for example, a lens, etc.) arranged on the optical axis of the treatment laser light and the aiming light in a direction that intersects the optical axis.

The control unit may move, at the irradiation position movement step, the irradiation position of the treatment laser light and the aiming light to an adjacent position that is offset in a forward direction defined by the irradiation plan by n intervals of the plurality of irradiation spots each time irradiation with the treatment laser light to n irradiation spots is completed. In this case, the irradiation position Al of the treatment laser light and the aiming light automatically moves to or near the target position at which the treatment laser light will be emitted next time (i.e., the position on the tissue where the irradiation spot is scheduled to be emitted next time) each time the treatment laser light is emitted without changing the relative position of the device and the angle of the contact lens by the operator. Thus, the accuracy and efficiency of treatment can be further improved.

The control unit may move the irradiation position of the treatment laser light and the aiming light to the adjacent position without performing irradiation with the treatment laser light upon receiving an instruction (hereinafter, referred to as a "skip instruction") for skipping irradiations with the treatment laser light to next n irradiation spots that are defined by the irradiation plan. For example, if a non-irradiation area where irradiation with the treatment laser light should not be performed exists, the operator inputs the skip instruction to skip the irradiation with the treatment laser light for the non-irradiation area. Then, the operator restarts treatment from the irradiation spot that is planned to be irradiated next. Therefore, it becomes easier for the treatment to proceed more smoothly.

The control unit may move, at the irradiation position movement step, the irradiation position of the treatment laser light and the aiming light in accordance with the instruction for moving the irradiation position of the treatment laser light and the aiming light in response to receiving this instruction. In this case, the operator can move the irradiation position AI to a desired position only by inputting the instruction for moving the irradiation position Al into the ophthalmic laser treatment device without changing the relative position of the device and the angle of the contact lens. Thus, it is easy to adjust the aiming point of the treatment laser light with a reduced amount of movement of the observation image.

It should be noted that, at the irradiation position movement step, the control unit may perform both the process for automatically moving the irradiation position based on the irradiation plan and the process for moving the irradiation position in response to receiving the instruction. In this case, when the irradiation position automatically moved based on the irradiation plan deviates from the position at which the treatment laser light is scheduled to be emitted next time, the operator can adjust the irradiation position by inputting the instruction for moving the irradiation position into the ophthalmic laser treatment device. Thus, the amount of movement of the observation image is further reduced, and the accuracy and efficiency of treatment can be further improved.

However, the ophthalmic laser treatment device may perform only one of the process for automatically moving the irradiation position based on the irradiation plan and the process for moving the irradiation position in response to receiving the inputted instruction. For example, when the irradiation position automatically moved based on the irradiation plan deviates from the position at which the treatment laser light is scheduled to be emitted next time, the operator can adjust the irradiation position by slightly changing at least one of the relative position of the device and the angle of the contact lens. Even in this case, the amount of movement of the observation image can be reduced.

The control unit may irradiate one irradiation spot with one shot of treatment laser light each time one irradiation execution instruction is input. In other words, "n = 1." It is assumed that a plurality of irradiation spots may be irradiated with treatment laser light each time one irradiation execution instruction is input. In this case, although the operator's workload is reduced and easier, the operator needs to accurately adjust all the multiple irradiation spots to the treatment target area and then input the irradiation execution instruction. On the other hand, when one shot of treatment laser light is emitted each time one irradiation execution instruction is input, the operator can adjust the target position of the treatment laser light for each of the multiple irradiation spots and then input the irradiation execution instruction. Thus, the position of each of the plurality of irradiation spots can be more accurately adjusted to the treatment target area. Further, it is easy to skip irradiation of non-irradiated areas with treatment laser light.

However, each time one irradiation execution instruction is input, the control unit may control the irradiation position moving unit based on the irradiation plan and emit treatment laser light multiple times at appropriate intervals to the multiple irradiation spots. In other words, "n ≥ 2." In this case, the operator's workload is further reduced.

At the guide display step, the control unit may control the display unit to display a spot interval guide indicating appropriate intervals between the plurality of irradiation spots on which irradiation with treatment laser light is scheduled to be performed in accordance with the progress of the irradiation plan. In this case, an operator can visually recognize the spot interval guide while observing the observation image of the tissue of the patient's eye and adjust the position of the irradiation spots for the treatment laser light on the tissue by referring to the recognized spot interval guide. Therefore, the operator can easily cause the interval between the irradiation spots, to which treatment laser light is applied, to be an appropriate interval.

The control unit may cause the intervals of at least some of the plurality of indicators in the spot interval guide to coincide with appropriate intervals of the plurality of irradiation spots. In this case, by emitting treatment laser light multiple times according to the intervals of the multiple indicators in the spot interval guide, the operator can easily bring spacings between the multiple irradiation spots close to the appropriate intervals. Therefore, it becomes easier to perform treatment in accordance with the treatment plan more appropriately.

However, it is also possible to change the displaying manner of the spot interval guide. For example, a part of the contact lens (for example, an inner wall, etc.) has a plurality of interval indicators (may be referred to as "indicators") placed at regular intervals to serve as a reference for the intervals between spots to which irradiation with treatment laser light is performed. The control unit may cause the intervals of at least some of the plurality of indicators included in the spot interval guide to coincide with the intervals of the interval indicators of the contact lens. In this case, the operator adjusts the aiming points while referring to the position and the direction of the interval indicators observed through the observation optical system and the position and the direction of the spot interval guide displayed on the display. Thus, the operator can appropriately adjust the aiming point for the treatment laser light.

Depending on the type of contact lens, the characteristics of the interval indicators provided on the contact lens (for example, the interval and number of interval indicators, etc.) may differ. Therefore, the control unit may change the displaying method of the spot interval guide (for example, the intervals and the number of the plurality of indicators in the spot interval guide) based on information regarding the contact lens being used. In this case, since an appropriate spot interval guide is displayed according to the interval indicator of the contact lens being used, the treatment can smoothly proceed.

The control unit may change the entire spot interval guide displayed on the display unit according to the magnification of the observation image of the observation optical system. That is, the control unit may increase the size of the spot interval guide as the magnification of the observation image presented by the observation optical system increases. In this case, even if the observation magnification is changed, appropriate intervals between the irradiation spots are appropriately indicated by the spot interval guide.

The guide may include a shape extending along an appropriate direction of reflection of the treatment laser light to the next irradiation spot by the reflective surface of the contact lens. The control unit may determine the angle of the guide displayed on the display unit according to the progress of the irradiation plan, and display the guide at the determined angle. In this case, the operator can easily adjust the rotation angle of the reflective surface of the contact lens by adjusting the reflection angle of the treatment laser light to follow the direction indicated by the guide. Furthermore, since the guide is displayed on the display unit at an appropriate angle according to the progress of the irradiation plan, the aiming point can be more appropriately adjusted.

It should be noted that if the interval indicators are formed on the contact lens, the operator can easily adjust the rotation angle of the reflective surface of the contact lens by adjusting the rotation angle so that the interval indicator is located ahead of the direction indicated by the shape of the guide.

The control unit may display ends of the plurality of indicators that are close to the aiming point for the treatment laser light to be located along a virtual curve line that extends approximately along the curve of the arc-shaped treatment site of the patient's eye. In this case, when the guide is set at an appropriate position with an appropriate orientation with respect to the next treatment target site, distances between one ends of the multiple indicators and the arc-shaped treatment target site are reduced. As a result, by referring to the displayed guide, the operator can more appropriately adjust the next aiming point of the treatment laser light.

Note that although the shape of the curve of the treatment target site of the patient's eye may be different from other patient's eyes, it does not have a significant change in each patient's eye. Therefore, the aiming point side end of each of the plurality of indicators may be displayed along a predetermined virtual curve line that is determined based on the average shape of the treatment target site.

The control unit may display at least one of the plurality of indicators in the guide by arranging the indicators along a straight line. For example, the control unit may display the indicators such that the centers of gravity of the indicators are arranged along a virtual straight line. Further, the control unit may display the indicators such that ends of the plurality of indicators that are away from the aiming point are arranged along a virtual straight line. In this case, the operator can proceed with the treatment more appropriately by matching the direction in which the plurality of indicators are arranged with the movement direction in which the aiming point of the treatment laser light is moved to the next aiming point. For example, the reflective surface of the contact lens may include an edge or the like perpendicular to a direction extending outward from the central axis of the lens. In this case, the operator aligns the direction of the edge of the contact lens reflected in the observed image with the linear direction in which the indicators are arranged, and aims the treatment laser light along the direction of the edge. In addition, even if the contact lens that was in contact with the patient's eye is once removed from the patient's eye, the operator can orient the edge of the contact lens in the observed image along the virtual straight line on which multiple indicators are lined up. It is also possible to easily restore the position and direction of the contact lens by adjusting the contact lens to the correct direction.

However, it is also possible to change the displaying manner of the guide. For example, the control unit may display a shape guide having a shape similar to the shape of the treatment target site where the multiple irradiation spots are scheduled to be irradiated with treatment laser light together with a spot interval guide (for example, multiple indicators arranged at appropriate intervals), or separately from the spot interval guide, on the display unit. The control unit may indicate, along the shape guide, a position (for example, a "next aiming indicator", etc., as will be described later) to be irradiated with the treatment laser light next time. In this case, the operator can easily adjust the aiming point to each of the plurality of irradiation spots by aligning the position and direction of the treatment target site appearing in the observation image with the position and direction of the shape guide superimposed on the observation image so that the shape of the shape guide matches the shape the treatment target site.

The control unit may display a next aiming indicator in the guide to set a next target position on the tissue to be irradiated next time. Each time the laser irradiation to n irradiation spots ("n" is a natural number equal to or more than 1) is completed, the control unit may shift the position of the next aiming indicator to an adjacent position that is shifted by one appropriate interval of the plurality of irradiation spots in the forward direction defined by the irradiation plan. In this case, the operator can more easily adjust the next aiming point by aligning the target position on the tissue at which the treatment laser light will be irradiated next time with the displayed next irradiation indicator.

When an instruction to omit irradiations with the treatment laser light to n irradiation spots defined in the irradiation plan (hereinafter, referred to as a "skip instruction") is input, the control unit may shift the next aiming point to an adjacent position that is offset by n appropriate intervals of the irradiation spots in the forward direction defined by the irradiation plan without performing irradiation with the treatment laser light. For example, if a non-irradiation area where irradiation with the treatment laser light should not be performed exits, the operator inputs the skip instruction to skip the irradiation with the treatment laser light for the non-irradiation area. Then, the operator restarts treatment from the irradiation spot that is planned to be irradiated next time. Therefore, it becomes easier for the treatment to proceed more smoothly.

It should be noted that, when the multiple indicators arranged corresponding to the multiple irradiation spots are included in the spot interval guide, the indicators other than the next aiming indicator are post-irradiation indicators corresponding to the spots to which the treatment laser light has been applied or pre-irradiation indicators corresponding to the spots to which the treatment laser light has not been applied. The control unit may display the next aiming indicator in a different manner (that is, in a manner distinguishable by the operator) from the post-irradiation indicator and the pre-irradiation indicator. In this case, the operator can easily recognize the position of the next aiming index. Furthermore, the control unit may display each of the next aiming indicator, the post-irradiation indicator, and the pre-irradiation indicator in a different manner. In this case, the operator can easily recognize the positional relationship between the next aiming indicator, the post-irradiation indicator, and the pre-irradiation indicator, making it easier to proceed with the treatment more smoothly.

However, there is no need to include the next aiming indicator in the guide. For example, the control unit may display multiple indicators on the display unit at predetermined intervals (e.g., the appropriate interval between the multiple irradiation spots, etc.) without changing the displaying manner. Even in this case, the operator can appropriately adjust each of the multiple irradiation spots on an appropriate position on the tissue by referring to the multiple indicators.

The shape of the treatment target site of the patient's eye onto which the treatment laser light is applied may be arcuate or annular. Here, an arc-shape region to which irradiations with the treatment laser light a predetermined number M of times while a displayed position and an angle of the guide on the display unit are fixed is defined as an irradiation section. Part or the entire treatment target site are formed of a plurality of irradiation sections. The control unit may rotate the entire guide in the forward direction by the angle corresponding to one irradiation section each time laser light irradiation onto all the plurality of irradiation spots in the irradiation section under treatment is completed. In this case, when treatment for one irradiation section is completed while the display position and angle of the guide are fixed, the displayed guide is rotated by an angle for the next irradiation section. Accordingly, irradiation with the treatment laser light onto each of the plurality of irradiation sections is automatically and appropriately assisted according to the progress of the irradiation plan.

The angle of the irradiation section can be selected as appropriate. For example, it is assumed that the range of R degrees (R<=360) in a ring-shaped or arc-shaped treatment target site (for example, trabecular meshwork, etc.) is divided into S irradiation sections, and the aiming point is adjusted within each irradiation section. In this case, the control unit may rotate the entire guide in the forward direction by the angle of one irradiation section (i.e., R/S degrees) each time laser light irradiation onto all the plurality of irradiation spots in a currently-treated irradiation section is completed. As a result, the laser irradiation is appropriately performed in each of the plurality of irradiation sections.

Note that when irradiations with the treatment laser light to n irradiation spots (n>=2) is performed by controlling the irradiation position moving unit each time the irradiation execution instruction is input, the specified number M of times the treatment laser light is emitted while the display position and the angle of the guide are fixed is a natural number equal to or more than n (M>=n).

When the control unit receives an instruction to rotate the entire guide to the next irradiation section before the laser irradiation onto all the plurality of irradiation spots in the irradiation section under treatment is completed, the entire guide may be rotated by an angle corresponding to a partial area of the currently-treated irradiation section where the treatment has been done. In this case, even if the treatment for the entire irradiation section is not completed, the operator can move forward to treatment for the next irradiation section by rotating the entire guide. Therefore, it becomes easier for the treatment to proceed more smoothly.

Note that the method for calculating the angle when rotating the guide to the next irradiation section can be selected as appropriate. For example, the angle between two adjacent irradiation spots is A degrees when viewed from the center of a circle through which multiple planned irradiation spots pass, and the number of the irradiation spots for which treatment has been done within the irradiation section under treatment is m. The angle corresponding to the range in which the treatment has been done within the irradiation section may be calculated by "A (degrees) x m (number)". The number of irradiation spots "m" for which treatment has been done may also include the number of irradiation spots for which irradiation with the treatment laser light was skipped in accordance with the above-described skip instruction.

When displaying, in the guide, the next aiming indicator for aiming the position of the next irradiation spot, the control unit may set one end of the guide in a direction opposite to the forward direction of treatment at the position indicated by the next irradiation indicator each time the guide is rotated to the next irradiation section. In this case, the position of the next aiming indicator is appropriately changed according to the progress of the irradiation plan, making it easier for the treatment to proceed more smoothly. Furthermore, when displaying the next aiming indicator, the post-irradiation indicator, and the pre-irradiation indicator in a distinguishable manner, the control unit may set all the indicators except the next irradiation indicator as the pre-irradiation indicators each time the plurality of indicators are rotated to the next irradiation section.

Note that it is also possible to change the specific method for rotating the entire guide (for example, a plurality of indicators in the spot interval guide). For example, the control unit may rotate the guide by the angle of the irradiation section each time a rotation instruction is input by a user (for example, an operator or an assistant). Further, the rotation angle of the guide may be set by a user.

Each time the guide is rotated to the next irradiation section, the control unit may move the irradiation position of the treatment laser light and the aiming light in a direction opposite to the forward direction defined by the irradiation plan. In this case, treatment for the next irradiation section can be started smoothly and easily.

In a second aspect of the present disclosure, an ophthalmic laser treatment device irradiates a tissue of a patient's eye with treatment laser light in response to receiving an irradiation execution instruction. The ophthalmic laser treatment device includes a treatment laser light source, an aiming light source, an irradiation position moving unit, an observation optical system, a display unit, and a control unit. The treatment laser light source is configured to emit treatment laser light. The aiming light source is configured to emit aiming light for an operator to recognize a scheduled irradiation position for the treatment laser light on the tissue. The irradiation position moving unit is configured to move, on the tissue, an irradiation position at which the treatment laser light and the aiming light are emitted. The observation optical system is configured to present an observation image of the tissue to the operator. The display unit is configured to display an image by superimposing the image on the observation image observed by the operator. The control unit executes an irradiation plan acquisition step and a guide display step. In the irradiation plan acquisition step, when irradiating the patient's eye with the treatment laser beam using a contact lens having a reflective surface that reflects the treatment laser light and the aiming light in a direction intersecting the optical axis, the control unit acquires an irradiation plan in which the order of irradiation of treatment laser light is set for a plurality of irradiation spots to be irradiated. At the guide display step, the control unit displays a guide on the display unit in accordance with a progress of the irradiation plan, wherein the guide is displayed to align a target position on the tissue with a next one of the plurality of irradiation spots at which the treatment laser light is scheduled to be emitted next time. At the guide display step, a non-adjacent position change step and a target position indicator display step are executed. At the non-adjacent position change step, when irradiations with treatment laser light to n irradiation spots are completed, the control unit changes the next target position, that is a target with which the target position on the tissue will be aligned next time, to a non-adjacent position different from the adjacent position. The adjacent position is a position that deviates from the irradiation spot at which irradiation with treatment laser light was completed the last time in the forward direction defined by the irradiation plan by "n" appropriate intervals of the irradiation spots. At the non-adjacent position change step, the control unit displays, when executing a target position indicator display step, a target position indicator on the display unit. The target position indicator indicates a position on the tissue at the adjacent position as the target position on the tissue with which the next aiming point of the guide will be aligned.

According to the ophthalmic laser treatment device in the present disclosure, the display unit displays a guide according to the progress of an irradiation plan. The guide is displayed to make a target position on the tissue at which treatment laser light is to be emitted match at least a next irradiation spot at which the treatment laser light will be emitted next time. Thus, an operator can visually recognize the guide while observing the observation image of the tissue of the patient's eye and adjust the position of the irradiation spots for the treatment laser light on the tissue by referring to the recognized guide. Thus, the operator can easily and appropriately irradiate each of the plurality of irradiation spots.

Also, in the ophthalmic laser treatment device according to the present disclosure, when the relative position of the device relative to the patient's eye (hereinafter simply referred to as the "relative position of the device") moves, the position of the optical axes of the treatment laser light and the aiming light also move. Thus, the irradiation position of the treatment laser light and the aiming light on the tissue move. Further, when the contact lens is rotated, the angle of the reflective surface of the contact lens changes, and thus the aiming point of the treatment laser light and the aiming light on the tissue moves. However, the amount of movement of the observation image observed by the operator increases as the amount of movement in the relative position of the device and the amount of rotation of the contact lens increases. Thus, it may be difficult to adjust the irradiation spot to an appropriate position on the tissue. Furthermore, the efficiency of treatment work also decreases as the frequency of changing the relative position of the device and the angle of the contact lens increases. In contrast, according to the ophthalmic laser treatment device in the present disclosure, the irradiation position moving unit moves the irradiation position of treatment laser light and the target light on the tissue of the patient's eye. Thus, the operator can easily adjust the arrangement of the irradiation spot on the tissue while the amount of movement of the observed image is reduced. The amount of operation performed by the operator can be also reduced. Accordingly, it is possible to appropriately support treatment with treatment laser light when a contact lens having a reflective surface is used.

On the other hand, the range within which the operator can observe the observation image by the observation optical system and the range in which the irradiation position (hereinafter, referred to as an "irradiation position of light") of the treatment laser light and the target light can be moved by the irradiation position moving unit are both limited. Thus, when the arrangement of the multiple irradiation spots on the tissue is adjusted by the guide that is superimposed on the observation image while the observation image is fixed, the next aiming point indicated by the guide may deviate from the observed image or the irradiation position of the light may deviate from the movable range. In order to maintain the next aiming point indicated by the guide within the range of the observation image and maintain the irradiation position of the light within a movable range, it is necessary for the control unit to move the next aiming point to the non-adjacent position (for example, a direction opposite to the planned treatment direction, etc.) different from the adjacent position (a position that is offset from the irradiation spot where irradiation with treatment laser light was completed the last time in the forward direction defined by the irradiation plan by n appropriate intervals of the irradiation spots). In this case, by changing at least one of the relative position of the device and the angle of the contact lens so that the target position on the tissue matches the next target position indicated at the non-adjacent position by the guide, it is possible to adjust the arrangement of the irradiation spots on the tissue while fixing the range of the observation image. However, when the next aiming point is moved to the non-adjacent position different from the adjacent position, the operator may lose sight of the target position on the tissue appearing in the observation image (the position at which the treatment laser light is emitted next time).

On the contrary, when the next aiming point indicated by the guide is moved to the non-adjacent position different from the adjacent position, the ophthalmic laser treatment device according to the present disclosure causes the display unit to display, as a target position on the tissue with which the next aiming point of the guide will be aligned next time, the target position indicator indicating the tissue at the adjacent position (that is, a position that would be indicated by the next aiming indicator assuming that the next aiming indicator is moved to the adjacent position). Thus, the operator can appropriately adjust the aiming point of the treatment laser light to the target position by recognizing the target position on the tissue by the target position indicator superimposed on the observation image and by aligning the recognized target position with the position indicated by the next aiming indicator. Accordingly, it is possible to appropriately support treatment with treatment laser light when a contact lens having a reflective surface is used.

The target position indicator may be a mark that surrounds the target position that is an adjacent position (that is, a position on the tissue that would be indicated by the next aiming indicator if the next aiming indicator was moved to the adjacent position. In this case, the operator can recognize the state of the tissue at the target position located inside the mark using the observation image on which the mark is superimposed. Thus, it is easier for the operator to appropriately set the tissue located inside the mark at the position indicated by the next aiming indicator.

However, it is also possible to change the displaying manner of the target position indicator. For example, the target position indicator may be a mark superimposed on the adjacent position (i.e., the target position). Even in this case, the operator can appropriately recognize the target position based on the state or the like of the tissue around the mark superimposed on the adjacent position (i.e., the target position).

The target position indicator may directly indicate the adjacent position (i.e., the target position). Also, the target position indicator may indicate the target position by indicating a position associated with the adjacent position (i.e., the target position). For example, the control unit may indicate the target position by indicating the position of the irradiation spot that was irradiated with treatment laser light just before. As described above, the adjacent position (the target position) is a position that deviates from the position irradiated with treatment laser light immediately before. The adjacent position deviates in the forward direction of the treatment defined by the irradiation plan by n appropriate intervals between the irradiation spots. Thus, the operator can properly recognize the target position (the adjacent position adjacent to the irradiation spot where the treatment laser light was irradiated immediately before) by recognizing, using the target position indicator, the position of the irradiation spot where the treatment laser light was emitted immediately before.

After the target position indicator is displayed on the display unit at the target position indicator display step, the control unit may remove the displayed target position indicator upon receiving a irradiation execution instruction (or after the irradiation execution instruction is input). When the position of the next aiming indicator indicated by the guide is moved to a position different from the adjacent position, the operator recognizes the target position on the tissue that will be irradiated with treatment laser light next time using the displayed target position indicator. Then, the operator sets the recognized target position on the tissue on the position of the next target indicator, and inputs the irradiation execution instruction into the ophthalmic laser treatment device. Therefore, after the irradiation execution instruction is inputted, there is no need to display the target position indicator since the target position on the tissue has been already set on the next aiming indicator. Thus, the control unit can erase the target position indicator at an appropriate timing by inputting the irradiation execution instruction.

The ophthalmic laser treatment device may also include an imaging unit for capturing an observation image on which an image is superimposed by the display unit. The control unit may present a reference image that is captured by the imaging unit when displaying the target position indicator at the target position indicator display step. In this case, the operator recognizes again the target position on the tissue indicated by the target position indicator by checking the provided reference image even after the operator changed at least one of the relative position of the device and the angle of the contact lens to align the target position on the tissue with the position of the next aiming indicator indicated by the guide (that is, even when the actual target position on the tissue is offset from the position indicated by the target position indicator that is fixedly displayed). Thus, the operator can appropriately adjust the target position on the tissue that is recognized by the target position indicator to the position indicated by the next aiming indicator.

Note that a method for presenting the reference image (the observation image) on which the target position indicator is superimposed to an operator can be selected as appropriate. For example, the control unit displays the reference image captured by the imaging unit in a part of the display area of the display unit where an image is superimposed on the observation image. In this case, by comparing the observation image of the tissue at this moment with the reference image at the time of displaying the target position indicator, the operator can easily align the target position with the position indicated by the next aiming indicator. Further, the control unit may display the reference image (the observation image) on which the target position indicator is superimposed on a separate display unit from the display unit that superimposes the image on the observation image

After presenting the reference image to the operator, the control unit may stops presenting the reference image when a irradiation execution instruction is inputted (or after the instruction was inputted). As described above, after the irradiation execution instruction is inputted, there is no need to present the reference image since the target position on the tissue has been already set on the next aiming indicator. Thus, the control unit can stop presenting the reference image at an appropriate timing by inputting the irradiation execution instruction.

The control unit may cause the position at which the target position indicator is superimposed and displayed by the display unit to follow the target position on the observation image as the observation image by the observation optical system moves. In this case, even if the target position on the tissue indicated by the target position indicator is not memorized, the operator can easily and appropriately set the target position to the position indicated by the next aiming indicator by considering the target position that is moving to follow the target position.

Note that a specific method for causing the superimposed position of the target position indicator to follow the target position on the observation image can be selected as appropriate. For example, the ophthalmic laser treatment device may include an imaging unit that captures an observation image of a tissue of the subject eye. The control unit may specify the target position on the observation image that is moving. The target position is specified by using known image processing (e.g., pattern matching) on the observation images that are continuously captured by the imaging unit. Then, the control unit may move the target position indicator to follow the specified target position.

However, the position of the displayed target position indicator may be fixed. Even in this case, the operator can appropriately adjust the aiming point to the target position by aligning the target position on the tissue recognized by the target position indicator with the position indicated by the next aiming indicator.

The control unit may move, at the irradiation position movement step, the irradiation position of the treatment laser light and the aiming light to an adjacent position that is offset in a forward direction defined by the irradiation plan by n intervals of the plurality of irradiation spots each time irradiations with the treatment laser light to n irradiation spots are performed. In this case, the irradiation position Al of the treatment laser light and the aiming light automatically moves to or near the target position at which the treatment laser light will be emitted next time (i.e., the position on the tissue where the irradiation spot is scheduled to be emitted next time) each time the treatment laser light is irradiated without changing the relative position of the device and the angle of the contact lens by the operator. Thus, the accuracy and efficiency of treatment can be further improved.

The control unit may display a next aiming indicator indicative of a next aiming point in the guide to set a next target position on the tissue to be irradiated with the treatment laser light next time. Each time the laser irradiation to n irradiation spots ("n" is a natural number equal to or more than 1) is executed, the control unit may shift the position of the next aiming indicator to an adjacent position that is shifted by one appropriate interval of the plurality of irradiation spots in the forward direction determined by the irradiation plan. In this case, the operator can more easily adjust the next aiming point by aligning the target position on the tissue at which the treatment laser light will be irradiated next time with the displayed next irradiation indicator.

The shape of the treatment target site of the patient's eye onto which the treatment laser light is applied may be arcuate or annular. Here, an arc-shape region to which irradiations with the treatment laser light a predetermined number M of times while a displayed position and an angle of the guide on the display unit are fixed is defined as an irradiation section. At the non-adjacent position change step, the control unit may rotate the entire guide in the forward direction by the angle of one irradiation section each time laser light irradiation onto all the plurality of irradiation spots in the irradiation section under treatment is completed. When the entire guide is rotated, the control unit may control the display unit to display the target position indicator indicating the target position on the tissue in the adjacent position (that is, the organizational position shown by the next aiming indicator when assuming that the next aiming indicator has moved to the adjacent position).

In this case, when treatment for one irradiation section is completed while the display position and angle of the guide are fixed, the displayed guide is rotated to have an angle corresponding to treatment for the next irradiation section. As a result, the next aiming point indicated by the guide is changed to the non-adjacent position different from the adjacent position. Furthermore, he target position indicator indicates a position on the tissue that would be indicated by the next aiming indicator if the next aiming indicator was moved to the adjacent position. The operator can smoothly start treatment on the next irradiation section by adjusting by recognizing the target position on the tissue by the target position indicator superimposed on the observation image and by aligning the recognized target position with the position indicated by the next aiming indicator. Accordingly, irradiation with the treatment laser light onto each of the plurality of irradiation sections is automatically and appropriately assisted according to the progress of the irradiation plan.

Each time the guide is rotated to the next irradiation section, the control unit may move the irradiation position of the treatment laser light and the aiming light in a direction opposite to the forward direction defined by the irradiation plan. In this case, treatment for the next irradiation section can be started smoothly and easily.

### <Embodiment>

Hereinafter, a plurality of typical embodiments according to the present disclosure will be described with reference to the drawings. An ophthalmic laser treatment device 1 in this embodiment can perform treatment of a patient's eye E by irradiating the patient's eye E with treatment laser light.

### <Overall configuration>

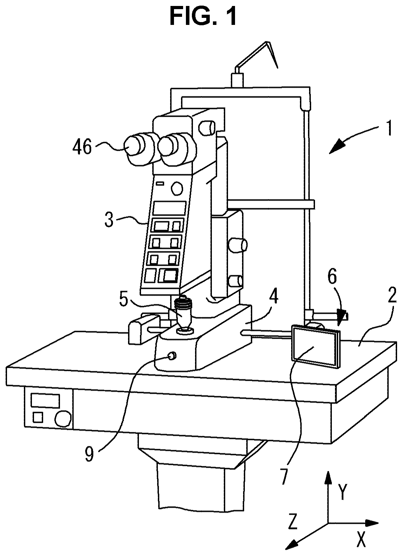

The configuration of the ophthalmic laser treatment device 1 will be described with reference to FIGS. 1 to 3. As shown in FIG. 1, the ophthalmic laser treatment device 1 according to this embodiment includes a table 2, a main body 3, and a control box 6. The main body 3 and the control box 6 are disposed on the table 2.

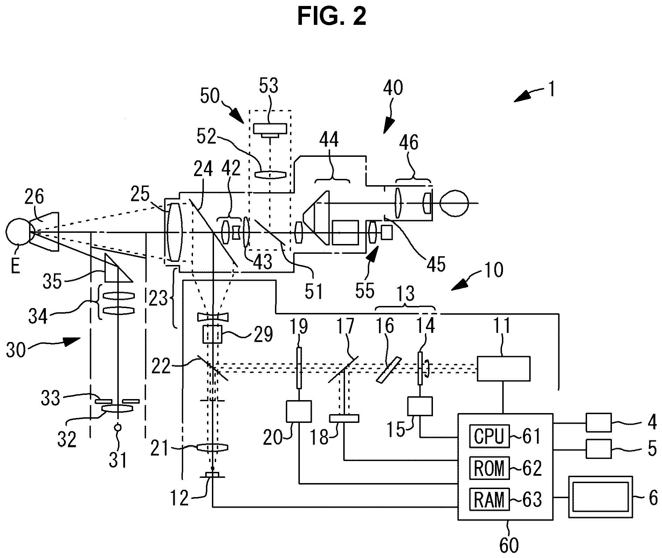

The main body 3 includes various components such as a laser irradiation optical system 10, an illumination optical system 30, an observation optical system 40, a display unit 50, an imaging unit 55, and a control unit 60 (see FIG. 2), which will be described later. Also, the main body 3 includes a base 4, a joystick 5 (an operation lever), and an operation unit 9 for an irradiation position. The base 4 is a displacement means having a displacement mechanism, and moves at least one of the laser irradiation optical system 10, the observation optical system 40, the display unit 50, and the imaging unit 55 in a vertical direction (Y direction in FIG. 1) and in a horizontal direction. (in X direction in FIG. 1), and in a front-back direction (in Z direction in FIG. 1). The displacement means changes a relative positional relationship between the patient's eye and the laser irradiation optical system 10 in an up-down direction, left-right direction, and front-back direction. The base 4 can further rotate at least one of the laser irradiation optical system 10, the observation optical system 40, the display unit 50, and the imaging unit 55 in the horizontal direction about an axis extending in the vertical direction. The operator operates the joystick 5 to move or rotate the laser irradiation optical system 10, the observation optical system 40, the display unit 50, and the imaging unit 55, thereby adjusting the observation position of the patient's eye E and the irradiation position of the laser beam (the treatment laser light and the aiming beam). Note that the joystick 5 in this embodiment (for example, the upper end of the joystick 5) is provided with an operation button for operation by an operator. In this embodiment, the operation button of the joystick 5 is used as trigger input means for inputting a trigger for emitting the treatment laser light. Note that a foot switch or the like operated by the operator's foot may be used as trigger input means for inputting a trigger for executing laser irradiation.

The control box 6 includes an external display 7 provided outside of the observation optical system 40 (see FIG. 2). The external display 7 can display various images. A touch panel type operation area is formed on a surface of the external display 7 of the control box 6. The control box 6 displays various parameters related to treatment on the external display 7 and receives inputs of various instructions from a user.

The irradiation position operation unit 9 is operated by an operator to input an instruction for moving the irradiation position of laser light (treatment laser light and aiming light) by an irradiation position moving unit 29, which will be described later. As an example, the irradiation position operation unit 9 in this embodiment is an operation lever, and the direction of movement of the irradiation position of the laser light is indicated according to the tilt of the operation lever. However, the specific configuration of the irradiation position operation unit may be changed. For example, multiple operation buttons, etc., for inputting an instruction for moving the irradiation position in any of the two-dimensional directions may be used as the irradiation position operation unit. The operator may input a skip instruction, which will be described later, by operating the irradiation position operation unit 9.

### <Laser irradiation optical system>

As shown in FIG. 2, the laser irradiation optical system 10 includes in this embodiment includes a treatment laser light source 11, an aiming light source 12, an energy adjustment unit 13, a beam splitter 17, a photodetector 18, a safety shutter 19, a collimator lens 21, a dichroic mirror 22, the irradiation position movement unit 29, an expander lens 23, a dichroic mirror 24, and an objective lens 25.

The treatment laser light source 11 emits treatment laser light for treating the tissue of the patient's eye E. As an example, in the treatment laser light source 11 in this embodiment, neodymium-doped YAG (yttrium aluminum garnet) crystal (Nd:YAG) is used as the laser rod. Further, a wavelength conversion element (not shown) is configured to convert infrared laser light (wavelength: 1064 nm) emitted by the treatment laser light source 11 into visible laser light (wavelength: 532 nm).

The aiming light source 12 emits aiming laser light (hereinafter, simply referred to as "aiming light") indicative of the position to be irradiated with the treatment laser light (that is, the position of the irradiation spot). In this embodiment, a light source that emits visible laser light with a wavelength of 635 nm (red) is used as the aiming light source 12. However, the wavelength of the aiming light may be changed as appropriate.

The energy adjustment unit 13 adjusts the amount of energy of the treatment laser light irradiated onto the tissue of the patient's eye E. The energy adjustment unit 13 in this embodiment includes a half-wave wavelength plate 14 and a polarizing plate 16. The half-wave plate 14 is rotated by a motor 15 about the optical axis of the treatment laser light. The polarizing plate 16 is arranged at Brewster's angle. The amount of energy of the treatment laser light is adjusted by the combination of the 1/2 wavelength plate 14 and the polarizing plate 16.

The beam splitter 17 reflects a portion of the treatment laser light toward the photodetector 18. The photodetector 18 detects an amount of energy of the treatment laser light by receiving the treatment laser light reflected by the beam splitter 17. The safety shutter 19 is moved between a position on the optical axis of the treatment laser light and a position outside of the optical axis by a shutter driving unit (for example, a solenoid) 20. When the safety shutter 19 is located on the optical axis of the treatment laser light, the safety shutter 19 blocks irradiation of the patient's eye E with the treatment laser light.

The collimator lens 21 converts the aiming light emitted by the aiming light source 12 into a parallel light beam. The dichroic mirror 22 has a light axis coaxial with the optical axes of the treatment laser light and the aiming light and is configured to multiplex the treatment laser light and the aiming light. The dichroic mirror 22 in this embodiment reflects the treatment laser light and transmits the aiming light, thereby combining the treatment laser light and the aiming light.

The irradiation position movement unit 29 moves, on the tissue of the patient's eye E, the irradiation position for the treatment laser light emitted by the treatment laser light source 11 and the irradiation position for the target light emitted by the aiming light source 12. The irradiation position moving unit 29 moves the irradiation positions of the treatment laser light and the aiming light by changing the position of the optical axes of the treatment laser light and the position of the aiming light with respect to the observation optical axis of the observation optical system 40. The irradiation position moving unit 29 in the present embodiment is arranged in a common optical path of the treatment laser light and the aiming light that are coaxed with each other by a dichroic mirror 22. As a result, the irradiation position moving unit 29 moves the irradiation position of the treatment laser light and the irradiation position of the aiming light on the tissue while keeping the treatment laser light and the aiming light coaxial. In this embodiment, a deflector (more specifically, a 2-axis galvanometer is used in this embodiment) that changes the deflecting direction of the treatment laser light and the aiming light is used as the irradiation position moving unit 29. However, the configuration of the irradiation position moving unit may be changed. For example, an irradiation position moving unit may change the irradiation position by moving an optical member (for example, a lens, etc.) arranged on the optical axis of the treatment laser light and the aiming light in a direction that intersects the optical axis. In this embodiment, the observation surface, the display 53, and the spot position (focal point) of the aiming light are optically conjugated. At the position on the observation surface, the observation optical axis, the center of the display area of the display 53, and the optical axis (i.e., the central axis) of the aiming optical system coincide. Further, the size of the guide 90 displayed on the display 53 and the amount of movement of the aiming light moved by the irradiation position moving unit 29 are calculated in consideration of observation magnification. The device is manufactured and the configuration of the device is adjusted so as to satisfy the conjugation relationship, the center coincidence, and the consideration of the observation magnification. Further, at the time of manufacturing and adjustment described above, the relationship between the displayable area of the display 53 and the displaceable range for the aiming light by the irradiation position moving unit 29 is stored in the storage medium in the device. Therefore, by controlling the irradiation position moving unit 29 based on data (program based on design values (theoretical values) or adjustment values stored during manufacturing adjustments) stored in the device in advance, the control unit is able to set (move) the spot center of the aiming light at an arbitrary coordinate position (within the displaceable range as described above) of the display 53 without performing image detection. The aiming light may also be moved in any direction by one spot. In this way, the irradiation position moving unit 29 in this embodiment performs control by considering and relating the characteristics of the observation optical system and the characteristics of the display 53.

The expander lens 23 expands the beam of laser light (treatment laser light and aiming light) that is combined by the dichroic mirror 22 and passed through the irradiation position moving unit 29. The laser beam expanded by the expander lens 23 is reflected by the dichroic mirror 24 and transmitted through the objective lens 25. In this embodiment, the laser light transmitted through the objective lens 25 is irradiated onto the tissue of the patient's eye E via the contact lens 26 attached to the patient's eye E. The dichroic mirror 24 reflects almost all the reflected light of the treatment laser light reflected by the patient's eye E so that the reflected light is less likely to enter the operator's eye. Note that the laser irradiation optical system 10 may be provided with a mechanism for adjusting the spot size of the laser light irradiated onto the tissue.

### <Illumination optical system>

The illumination optical system 30 illuminates the observation site including the treatment target site of the tissue. The illumination optical system 30 in this embodiment includes a lamp 31, a lens 32, an aperture 33, a lens group 34, and a prism 35. For example, a white light emitting element or the like may be used as the lamp 31. Note that the illumination optical system 30 may include a slit plate or the like for illuminating the observation site with slit light.

### <Observation optical system>

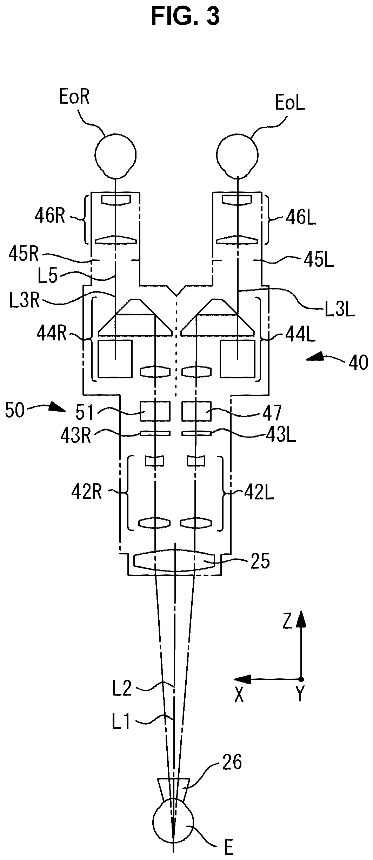

As shown in FIGS. 2 and 3, the observation optical system 40 is observation means for allowing the operator to observe the patient's eye E, and includes an optical axis L3 (the light axes L3R, L3L shown in FIG. 3). As shown in FIG. 3, the observation optical system 40 in this embodiment has an optical axis L3R for presenting an observation image to the operator's right eye EoR and an optical axis L3R for presenting an observation image to the operator's left eye EoL. and an optical axis L3L. The observation optical system 40 in this embodiment may be binoculars. The observation optical system 40 in this embodiment includes an objective lens 25, a variable magnification optical system 42 (42R, 42L), a protective filter 43 (43R, 43L), a half mirror 47, an erecting prism group 44 (44R, 44L), and a visual field aperture 45 (45R, 45L), an eyepiece 46 (46R, 46L), and the like. The operator looks through the eyepiece 46 and checks the observation image of the tissue of the patient's eye E, the spot of the aiming light (in other words, the reflected light (return light) of the aiming light reflected by the patient's eye E), and the images displayed by the display unit 50. In this embodiment, an observation surface (object surface) disposed at the tip of the objective lens 41 and a field stop 45 disposed inside the device are in an optically conjugate positional relationship via the objective lens 41. That is, at the position of the field stop 45, an observation image of the patient's eye E is formed as an aerial image (a virtual image). Note that in this embodiment, the magnification of the observation image observed by the operator is changed by the variable magnification optical system 42. The observation optical system 40 is provided with an encoder (not shown) for obtaining the magnification of the observation image by the variable magnification optical system 42.

### <Display>

The display unit 50 (see FIG. 2) displays images by superimposing the images on an observation image created by the observation optical system 40 for an operator. As shown in FIG. 2, the display unit 50 is disposed in the observation optical system 40 and displays images to the operator via the eyepiece 46. The display unit 50 includes a display 53, a lens 52, and a half mirror 51. Various images are displayed on the display 53. In this embodiment, an LCD (with a backlight) is used as the display 53. Specifically, in this embodiment, a color LCD capable of displaying 1600 (H) x 1200 (V) is used as the display 53. As shown in FIG. 3, the half mirror 51 is arranged on the optical axis L3R. Specifically, the half mirror 51 is arranged between the protective filter 43R and the erecting prism group 44R.

Presentation light (display light) emitted from the display 53 travels along the optical axis L5 (see FIG. 3). Specifically, the presentation light emitted from the display 53 passes through the lens 52 and is then reflected by the half mirror 51 in a direction toward the erecting prism group 44R. The half mirror 51 in this embodiment is combining means for combining the optical observation image observed by the observation optical system 40 and the image displayed on the display 53. The half mirror 51 in this embodiment has an axis coaxial with the optical axis L5 and the optical axis L3R. The presentation light reflected by the half mirror 51 passes through the erecting prism group 44R, the field stop 45R, and the eyepiece lens 46R in this order, and is focused on the fundus of the operator's eye looking through the eyepiece lens 46R. The display unit 50 in the present embodiment functions as a so-called head-up display (HUD).

In this embodiment, the display 53 and the field stop 45R are in an optically conjugate positional relationship. In other words, the image displayed on the display 53 is formed as an aerial image at the position of the field stop 45R. Note that the method of displaying the image to the operator through the eyepiece lens 46 is not necessarily limited to the method exemplified in this disclosure. For example, an LCD (liquid crystal panel) without a backlight is arranged as a display unit at the position of the field stop 45R (i.e., on the optical axis L3R), and the control unit 60 may control the transmittance of each cell constituting the LCD to present presentation information to the operator. Further, the display unit may be configured to project an image onto the retina of the operator's eye by scanning the retina with light rays. Further, the ophthalmic laser treatment device 1 captures an image of a tissue of the patient's eye E by the imaging unit 55, which will be described later, and displays the captured image on a display device (for example, a control box 6, etc.) so that an operator can observe the observation image of the tissue of the patient's eye E. In this case, the display device may superimpose other images on the observation image. In other words, the display device may provide both the function of the observation optical system for the operator to observe the observation image of the tissue of the patient's eye E and the function of the display unit for displaying the image on the observation image.

In this embodiment, the center of the display area in the display unit 50 is aligned with (i.e., matches) the observation optical axis of the observation optical system 40. Therefore, an image is displayed in the display area of the display unit 50 with the observation optical axis of the observation optical system 40 as a reference. Therefore, the operator can appropriately perform treatment while recognizing the image presented at an appropriate position in the observation field through the eyepiece lens 46.

### <Imaging unit>

As shown in FIG. 2, the imaging unit 55 captures an observation image of the patient's eye E, etc. More specifically, the imaging unit 55 in the present embodiment captures an observation image on which an image is superimposed by the display unit 50. Detailed illustrations are omitted, but the imaging unit 55 in this embodiment is provided with a half mirror, an imaging lens, and an imaging element. The half mirror is disposed on either a left-side observation optical path or a right-side observation optical path disposed in the observation optical system 40. Light that enters the half mirror from the observation site or the like via the objective lens 25 is reflected by the half mirror, and enters the imaging element via the imaging lens. As a result, an observation image is captured by the imaging optical system.

### <Control unit>

The control unit 60 manages various controls of the ophthalmic laser treatment device 1. The control unit 60 in this embodiment includes a CPU (processor) 61, a ROM 62, a RAM 63, a nonvolatile memory 65, and the like. The CPU 61 controls each component of the ophthalmic laser treatment device 1 . The ROM 62 stores various programs, initial values, and the like. The RAM 63 temporarily stores various information. The nonvolatile memory 65 is a non-transitory storage medium that is configured to retain stored contents even if the power supply turned off. For example, a USB memory that is removably attached to the control unit 60, a flash ROM built into the control unit 60, or the like may be used as the nonvolatile memory 65. In this embodiment, the control unit 60 is connected to the base unit 4, the joystick 5, the control box 6, the treatment laser light source 11, the irradiation light source 12, the motor 15, the photodetector 18, the shutter drive unit 20, the lamp 31, the display device. 53, and the imaging unit 55.

Note, in this disclosure, the term "processor" refers to one or more hardware processors configured to execute program code (i.e., one or more instructions of a program) in a program. In other words, a "processor" is a hardware device that is capable of executing one or more programmed processes. For example, a "processor" is a general-purpose or special-purpose processor, and may be at least any of a CPU, microprocessor, GPU, or DFP (Data Flow Pro).

In this disclosure, the term "memory" refers to one or more hardware memories that are non-transitory tangible storage media configured to store computer program code and/or data accessible to a processor. "Memory" may be implemented with memory technologies such as SRAM, SDRAM, non-volatile/flash type memory, or other types of memory. The computer program code constituting the program is stored on a memory and executed by a processor to cause the ophthalmic laser treatment device 1 to realize various functions.

In the present disclosure, the term a "circuit" indicates a logic circuit as a single or multiple hardware, and is configured to cause the ophthalmic laser treatment device 1 to perform various functions. In other words, the "circuit" refers to one or more non-programmable devices. For example, the "circuit" may be a custom IC or the like designed to be unprogrammable for a specific application.

In the present disclosure, at least one of a circuit and a processor having a memory storing computer program code causes the ophthalmic laser treatment device 1 to perform functions. The expression "at least one of a circuit and a processor" should be interpreted as discrete (i.e., logical sum), and should not be interpreted as at least one circuit and at least one processor.

### <Treatment manner for trabecular meshwork>

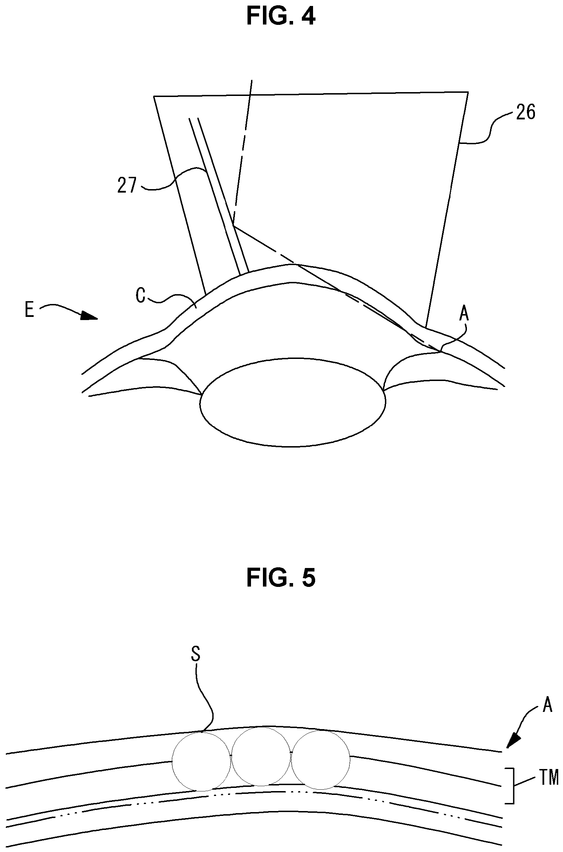

An example of a treatment manner for trabecular meshwork performed by the ophthalmic laser treatment device 1 in this embodiment will be described with reference to FIGS. 4 and 5. In this embodiment, the trabecular meshwork, which is a ring-shaped (partially, arc-shaped) tissue of the patient's eye E, is the target site for treatment. For example, a selective laser trabeculoplasty (SLT) is a treatment method that involves irradiating the trabecular meshwork at the angle of the patient's eye E with treatment laser light in order to increase the drainage of aqueous humor in the patient's eye E. Further, micropulse laser trabeculoplasty (MLT) is a treatment method for lowering intraocular pressure in a patient's eye by irradiating the trabecular meshwork with laser light having lower energy than laser used in SLT and activating the trabecular meshwork cells to improve aqueous humor flow. Note, SLT and MLT are less likely to leave irradiation marks by the treatment laser light. In SLT and MLT, the treatment laser light is irradiated multiple times over the entire circumference or part of the annular-shape trabecular meshwork. Note that SLT is based on Selective Phothermolysis theory. As an example other than SLT and MLT, minimally invasive cell-selective laser treatment (see WO 2017/174785 A1 for details, which is incorporated herein by reference) has been known where pulses around 200ns forming square spots with a side length of 50 µm in 1 sequence are used for scanning to form the equivalent of spots with a diameter of 400 µm. These have in common adjacent irradiation along the trabecular meshwork and difficulty in leaving treatment marks. In the following description, SLT is used as an irradiation method.

As shown in FIG. 4, in the treatment of trabecular meshwork in this embodiment, the contact lens 26 is attached to the cornea C of the patient's eye E. As the contact lens 26, for example, a gonioscope or a Goldmann three-sided mirror for observing the corner A of the patient's eye E may be used. The contact lens 26 is provided with a reflective surface (i.e., a reflective mirror) 27. The corner A is observed through the reflective surface 27. Therefore, as shown in FIG. 5, in this embodiment, the entire corner A (the entire circumference) cannot be observed at the same time, but a portion of the corner A is observed in a fan shape (a sector shape). The range of the fan-shaped portion of the annular corner A that can be observed through the reflective surface 27 falls within an angular range of less than 180 degrees with the center of the annular corner A as a reference. In addition, the reflective surface 27 of the contact lens 26 reflects the treatment laser light and aiming light in a direction intersecting the optical axis extending from the objective lens 25 (see FIGS. 2 and 3) toward the patient's eye E, thereby, irradiating the trabecular meshwork TM, which is the treatment target site, is irradiated with the treatment laser light and the aiming light. That is, in this embodiment, the trabecular meshwork is treated by irradiating the trabecular meshwork TM at the corner A with the treatment laser light, as illustrated in FIG. 5.

By adjusting the rotation angle of the contact lens 26 (that is, the angle of the rotation direction about the axis of the contact lens 26), the operator can adjust a reflective direction of the treatment laser light by the reflective surface 27 of the contact lens 26 as viewed from the operator's line of sight in the observation optical system 40. In addition, the operator may operate the joystick 5 to move the base 4 to move the relative position of the ophthalmic laser treatment device 1 (more specifically, the laser irradiation optical system 10), whereby the aiming point of the treatment laser light and the aiming light with respect to the tissue of the patient's eye E can be adjusted to a target spot S for treatment. Note that when the rotation angle of the contact lens 26 and the relative position of the device are changed, the observation image observed by the operator via the observation optical system 40 is also moved. Furthermore, in this embodiment, the operator can also move the irradiation position of the treatment laser light and the aiming light without moving the observation image by operating the irradiation position operation unit 9. After the adjustment of the aiming point is completed, the operator inputs an instruction to emit the treatment laser light by operating the operation button of the joystick 5 or the foot switch, thereby irradiating the spot S with the treatment laser light. Note that in SLT, treatment laser light is emitted with a lower output (energy and irradiation time) than in argon laser trabeculoplasty (ALT) in order not to cause thermal degeneration of the tissue. Therefore, it is difficult for the operator to visually recognize changes in the condition of the treated area (for example, treatment scars, etc.) before and after the surgery. In addition, as SLT and MLT procedures, there has been known that the spot size of the treatment laser light and the aiming light is fixed to a predetermined size (for example, 400 µm, etc.), and the treatment laser light is intermittently emitted so that multiple irradiation spots are adjacent to each other along the trabecular meshwork TM. FIG. 5 schematically shows part of a procedure in which treatment laser light is intermittently emitted so that a plurality of irradiation spots are arranged adjacent to each other in a circumferential direction. Note that in SLT and MLT, it is difficult to visually observe treatment scars. That is, the irradiated spot (spot S) irradiated with the treatment laser light is not visually observed by the operator as shown in FIG. 5.

### <Contact lens>

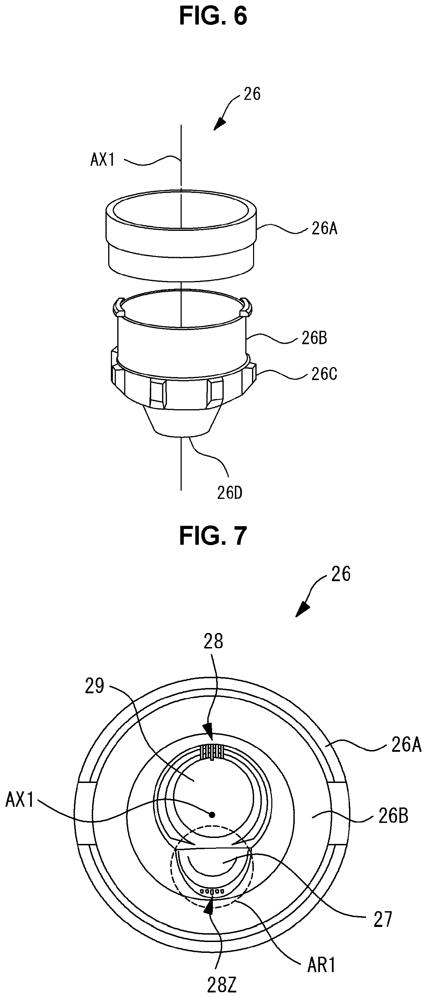

An example of the contact lens 26 used in the treatment of trabecular meshwork in this embodiment will be described with reference to FIGS. 6 and 7. The contact lens 26 shown in FIGS. 6 and 7 is a partially-rotatable-type lens. In the partially-rotatable-type lens, each time the lens is operated by the user's (operator's) finger, the portion including the reflective surface 27 rotates by a specified angle with respect to a grip 26A held by the user. That is, when the user performs one operation with a finger, a portion including the reflective surface 27 rotates in the circumferential direction about the axis AX1 independently of other portions.

Specifically, the contact lens 26 shown in FIG. 6 includes an annular grip 26A and a rotation base 26B. The grip 26A has a substantially cylindrical shape and is gripped by a plurality of fingers of the user. The rotation base 26B has a cylindrical portion with an outer diameter approximately equal to the inner diameter of the substantially cylindrical grip 26A. The tip of the rotation base 26B (a lower side of the paper in FIG. 6) serves as a contact portion 26D that comes into contact with the patient's eye. A transparent window 29 (see FIG. 7) is formed in the contact portion 26D. A reflective surface 27 (see FIG. 7) is fixed inside the rotation base 26B. The grip 26A is attached to the outer side of the cylindrical portion of the rotation base 26B. The rotation base 26B can rotate relative to the grip 26A. A plurality of protrusions 26C that protrude outward are provided on a portion of the outer periphery of the rotation base 26B that is exposed to the outside when the grip 26A is attached. In this embodiment, a plurality of (10 in this example shown in FIG. 6) protrusions 26C are provided at equal intervals in the circumferential direction at each of the aforementioned predetermined angles (36 degrees in the example shown in FIG. 6). The user can rotate the rotation base 26B provided with the reflective surface 27 with respect to the grip 26A by placing fingers on at least one of the protrusions 26C. Specifically, the user grips and fixes the grip 26A with multiple fingers (for example, indicator finger and thumb) and rotates only the rotation base 26B by hooking the remaining fingers (for example, the middle finger) on the protrusion 26C (the rotation axis is axis AX1). Therefore, as compared to the reflective surface that is rotated by rotating the entire contact lens, fewer finger movements are required during operation. As a result, the contact lens 26 is easily held in a stable manner even when the reflective surface 27 is rotated.

In the contact lens 26 shown in FIG. 6, the shape is designed so that the rotation angle of the rotation base 26B due to one rotation operation by the user's finger has a specified value (a specified angle). As an example, in the contact lens 26 shown in FIG. 6, the rotation base 26B rotates 36 degrees with respect to the grip 26A by a single operation. Therefore, the rotation base 26B rotates with respect to the grip 26A once by rotating ten times in the same direction. It should be noted the specified angle can be changed. For example, a partially rotatable lens in which the rotating base rotates 45 degrees with respect to the grip by a single operation may be used.

FIG. 7 is a view of the contact lens 26 on a side opposite to the patient's eye E. As shown in FIG. 7, interval indicators 28 is formed on a part of the inner wall of the contact lens 26 (in the example shown in FIG. 7, the inner wall of the rotation base 26B). The plurality of interval indicators 28 are arranged at regular intervals to serve as a guide for intervals between the spots that are irradiated with treatment laser light. In the example shown in FIG. 7, five interval indicators that extend linearly are arranged at equal intervals. The interval indicators 28 are formed on an inner wall of the contact lens 26 opposite to a side of the inner wall where the reflective surface 27 is provided. When the contact lens 26 is viewed from a side of the contact lens 26 opposite to the patient's eye E, a reflected image 28Z of at least one of the interval indicators 28 is reflected on the reflective surface 27. Further, during the treatment, a reflected image of the treatment target site (in this embodiment, the trabecular meshwork) of the patient's eye E is also reflected (in other words, viewed) on the reflective surface 27. Furthermore, a reflected image of the aiming light irradiated onto the treatment target site is also reflected on the reflective surface 27 . Therefore, while recognizing the positional relationship between the treatment target site reflected on the reflective surface 27 and the aiming light, the operator can adjust the aiming point of the treatment laser light to each of the plurality of spots in the treatment target site one by one using the reflected image 28Z of the interval indicators 28 as a guide. Note that the diagram of the observation field illustrated in each drawing in the present disclosure is a diagram showing an area corresponding to the area AR1 shown in FIG. 7 that is observed in an enlarged manner. Note that the reflective surface 27 is formed with a size that does not cause the reflected image (reflected image 28Z) of the interval indicators 28 to be missing. In this embodiment, the irradiation of the treatment laser light to the entire circumference of the trabecular meshwork TM is performed by rotating the reflective surface 27 about the axis AX1 one time while maintaining a state where the center of the pupil of the patient's eye E and the center of the light-transmitting window 29 are substantially aligned with each other regardless of the type of the contact lens 26 (for example, regardless of whether the interval indicators 28 are provided and whether the contact lens 26 is a partially rotatable lens). At this time, the area AR1 is made to follow the displacement of the reflective surface 27 as appropriate. Therefore, the operator operates the joystick 5 while irradiating the entire periphery of the trabecular meshwork TM with the treatment laser light to rotate the region AR1 about the axis AX1.

Note that the contact lens 26 shown in FIGS. 6 and 7 is only one of contact lenses that can be used in treatment with the ophthalmic laser treatment device 1 in this embodiment. Therefore, it is also possible to use a contact lens different from the contact lens 26 shown in FIGS. 6 and 7 for treatment in the ophthalmic laser treatment device 1 in this embodiment. For example, it is possible to use a contact lens for treatment that includes the reflective surface 27 and the interval indicators 28 but is not a partially rotatable lens (that is, the reflective surface 27 is rotated by rotating the entire lens). It is also possible to use a partially rotatable lens without the interval indicators 28 for treatment. It is also possible to use a contact lens that does not include the interval indicators 28 and is not a partially rotatable lens.

In addition, if a contact lens that requires the entire body to be rotated in order to rotate the reflective part is used, the operator grasps the contact lens with multiple fingers and rotates the entire contact lens while keeping the contact portion in contact with the patient's eye E. In this case, there is a limit to the angle at which the operator can rotate the entire contact lens without changing his/her grip (without having to re-grip the contact lens). Furthermore, when the operator changes his/her grip of the contact lens, the positional relationship between the patient's eye E and the laser irradiation optical system 10 may easily change. On the contrary, in the partially rotating contact lens 26 illustrated in FIGS. 6 and 7, the reflective surface can be rotated with each specified angle without changing operator's grip of the contact lens.

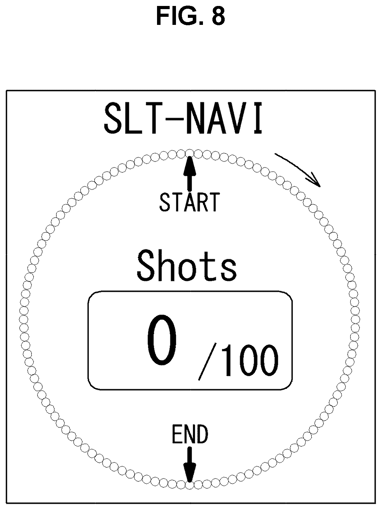

One example of an irradiation plan for treatment laser light will be described with reference to FIG. 8. FIG. 8 is a diagram showing an example of the irradiation plan displayed on a control box. In this embodiment, the irradiation plan is prepared by operating the control box 6. In the irradiation plan, when the patient's eye E is irradiated with the treatment laser light using the contact lens 26 having the reflective surface 27, an irradiation order for a plurality of irradiation spots to which the treatment laser light is to be irradiated is defined. In the irradiation plan in this embodiment, an irradiation range of the treatment laser light (that is, a range in a circumferential direction in which a plurality of irradiation spots are arranged) in an annular treatment target site (in this embodiment, a trabecular meshwork TM), the irradiation spot to which the treatment laser light is emitted first (i.e., the irradiation spot at the "START" position shown in FIG. 8), the number of irradiation spots (the number of denominators of "Shots" shown in FIG. 8), and the irradiation order (including an irradiation direction) of the treatment laser light for each of the irradiation spots. In FIG. 8, an arrow in a circumferential direction indicates the direction of the irradiation order. In this embodiment, each time a single treatment laser light is emitted, the irradiation spot adjacent to the irradiation spot where the irradiation has been completed becomes a next irradiation spot to which the treatment laser light will be emitted next, and "1" is added to the numerator of "Shots" shown in FIG. 8. In addition, each time one treatment laser light is emitted, a displaying manner of a spot (an irradiated spot) corresponding to a spot where an irradiation has been completed among multiple irradiation spots arranged in an arc shape or an annular shape (the annular shape in FIG. 8) is changed to be different from that of other spots. The multiple irradiations with the treatment laser light are sequentially performed in a clockwise or counterclockwise direction.

In the present embodiment, the direction in which the treatment target site to which the treatment laser light is emitted is actually located and the direction in which the reflective surface 27 of the contact lens 26 is directed to observe the treatment target site are opposite directions relative to the optical axis of the observation optical system 40. In this embodiment, the direction in which the treatment laser light should be emitted is indicated by the direction in which the reflective surface 27 is directed. However, the direction in which the treatment laser light should be emitted may be indicated by the direction in which the treatment target site is actually located. In addition, in another embodiment as described later, information regarding the contact lens used in treatment (for example, at least one of information indicating whether the contact lens is a partially rotatable lens, information on the specified angle for the partially rotatable lens, information indicating whether the interval indicators 28 are formed on the contact lens) may be included in the irradiation plan.

As an example, in this embodiment, the operator specifies an irradiation plan area and the number of irradiation spots at once by specifying either an "entire circumferential" mode or a "half circumferential" mode. Specifically, in this embodiment, there is the "entire circumferential" mode in which the treatment laser light is emitted to multiple spots over the entire circumference of the angle A, and the "half circumferential" mode in which the treatment laser light is emitted to multiple spots over the half of the circumference of the angle A. The default value of the number of spots to be irradiated in the "entire circumferential" mode is set to 100. The default value of the number of spots to be irradiated in the "half circumferential" mode is set to 50. When the "entire circumference" mode is selected by the operator, the control unit 60 sets the area covering the entire circumference of the corner A as the planned irradiation area, and sets "100" as the number of spots scheduled to be irradiated. In the example shown in FIG. 8, the "entire circumferential" mode is selected. Further, when the "half circumferential" mode is selected by the operator, the control unit 60 sets the area covering half the circumference of the corner A as the irradiation planed area, and sets "50" as the number of spots to be irradiated. In this embodiment, when the "half circumferential" mode is selected, the operator operates the touch panel to specify the "upper half" "lower half" "right half" "left half", etc. of the angle A to specify the detailed position of the area to be irradiated in the corner A. The control unit 60 sets the planned irradiation area based on the input result.

It should be noted that the method for accepting the designation of the irradiation planned area and the number of irradiation spots may be changed. For example, the ophthalmic laser treatment device 1 may prepare in advance modes other than the "entire circumferential" mode and the "half circumferential" mode. The control unit 60 may accept a designation of an angle (within a range of 360 degrees) from an operator and set a region corresponding to the designated angle as the planned irradiation region. The control unit 60 may allow the operator to directly input the number of spots to be irradiated, and may set the input number of spots to be irradiated regardless of the selected mode. The control unit 60 may change the number of spots to be irradiated, which is predetermined for each mode, in accordance with an operation by the operator. The control unit 60 may allow the operator to input the interval between two adjacent spots, and also calculate the number of spots to be irradiated according to the parameters of the planned irradiation area (for example, the length in the circumferential direction of the treatment target site) and the input interval between the spots..

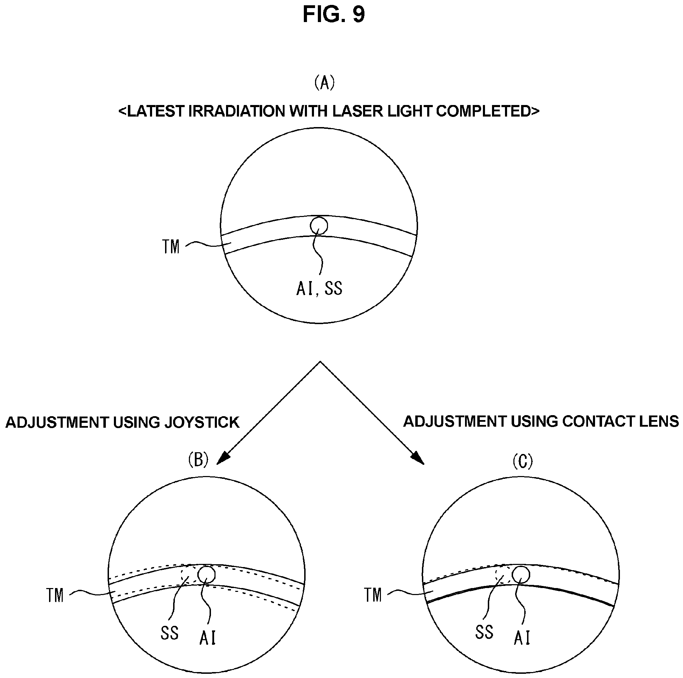

A method for adjusting the aiming point of the irradiation spot will be described as a comparative example with reference to FIG. 9. FIG. 9 (A) shows a state in which the latest irradiation with the treatment laser light has been completed. In the state of FIG. 9 (A), the position of the irradiated spot SS where an irradiation with the treatment laser light has been completed matches the aiming point (i.e., the position of the aiming light) Al. From the state shown in FIG. 9 (A), the operator changes the aiming point Al (that is, the aiming point of the irradiation spot) to a position of the next irradiation spot (in the example shown in FIG. 9, a position right next to the position of the irradiated spot SS).

FIG. 9 (B) shows a state where the aiming point is changed from the state shown in FIG. 9 (A) to the position of the next irradiation spot by operating the joystick 5 (that is, with the angle of the reflective surface 27 of the contact lens 26 fixed). In FIG. 9 (B), the position of the irradiated spot SS at the time of completion of the irradiation performed just before and the treatment target site (the trabecular meshwork TM in this embodiment) are schematically shown by dotted lines. However, in reality, since there is no treatment mark in the irradiated spot SS at the time of completion of the irradiation performed just before, it is difficult to accurately adjust the aiming point by operating the joystick 5.

In addition, FIG. 9 (C) shows a state where the aiming point is changed from the state shown in FIG. 9 (A) to the next irradiation spot position by rotating the reflective surface 27 of the contact lens 26 (that is, without operating the joystick 5). Further, in FIG. 9 (C), the position of the irradiated spot SS at the time of completion of the irradiation performed just before and the treatment target site are schematically shown by dotted lines. However, in reality, even in FIG. 9 (C), there is no treatment scar in the irradiated spot SS at the time of completion of the irradiation performed just before, and it is also difficult to accurately adjust the aiming point by rotating the reflective surface 27.

As described above, it can be seen that the observation image observed by the operator moves in both the case of operation of the joystick 5 (see FIG. 9 (B)) or the case of the rotation operation of the contact lens 26 (see FIG. 9 (C)). As the observation image moves frequently, it gets difficult to adjust the aiming point to an appropriate position on the tissue, and the efficiency of treatment work also decreases. In contrast, the ophthalmic laser treatment device 1 in the present embodiment is able to move the irradiation position of treatment laser light and the aiming light on the tissue of the patient's eye E by the irradiation position moving unit. Thus, the operator can easily adjust the arrangement of the irradiation spot on the tissue while the amount of movement of the observed image is reduced. Also, for example, the number of operations of the contact lens to move the observation image (i.e., change the aiming site) can be significantly reduced. Furthermore, the ophthalmic laser treatment device 1 in this embodiment assists the operator in adjusting the aiming point by displaying the guide on the display unit 50.

### <First embodiment>

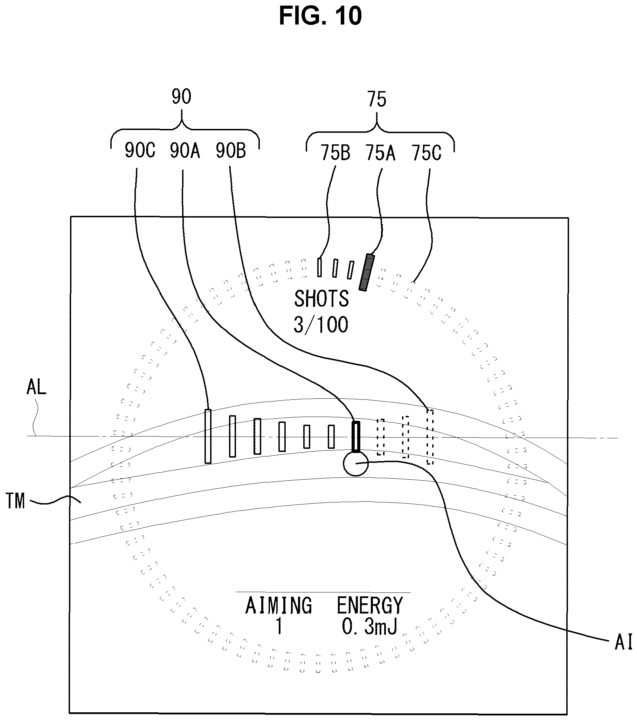

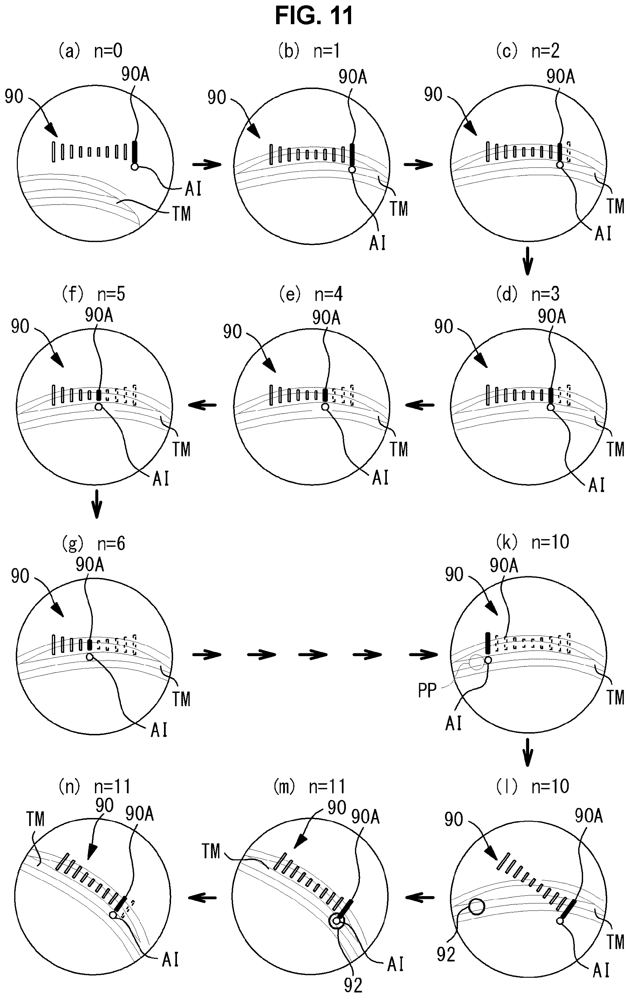

A treatment control process executed by an ophthalmic laser treatment device 1 according to a first embodiment will be described with reference to FIGS. 10 to 12. The ophthalmic laser treatment device 1 according to the present embodiment displays a guide 90 according to the progress of the irradiation plan. The guide 90 is displayed to make a target position on the tissue at which treatment laser light is to be irradiated match the irradiation spot at which the treatment laser light is emitted. Further, the ophthalmic laser treatment device 1 in the present embodiment is able to move the irradiation position of treatment laser light and the target light on the tissue by the irradiation position moving unit 29. Thus, even when the rotation angle of the contact lens 26 and the relative position of the device are maintained (that is, even when the observation image on the display unit 50 is fixed), the irradiation position of the light can be moved on the tissue. Furthermore, the ophthalmic laser treatment device 1 displays a target position indicator 92 (see FIG. 11 (l), (m)) indicating a target position PP (see FIG. 11 (k)) to be irradiated with treatment laser light next time on the display unit 50. In this embodiment, the center of the display area in the display unit 50 is aligned with (i.e., matches) the observation optical axis of the observation optical system 40. Also, in this embodiment, a single shot of treatment laser light is performed each time a treatment laser light execution instruction is input by an operator.

First, with reference to FIG. 10, one example of the operator's observation field of view during treatment by the ophthalmic laser treatment device 1 according to the first embodiment will be described. As shown in FIG. 10, the display unit 50 in this embodiment superimposes images on an observation image observed by an operator using the observation optical system 40. In the example shown in FIG. 10, the ophthalmic laser treatment device 1 superimposes an outer circumferential indicator 75, the guide 90, etc. on an observation image of a tissue (including trabecular meshwork TM) of a patient's eye.

The outer circumferential indicator 75 indicates at least one of a rough rotation angle of the reflective surface of the contact lens suitable for the progress of the irradiation plan (that is, a guiding direction in which the reflective surface 27 is oriented about the central axis AX1 of the contact lens 26) and a rough direction in which the irradiation spot to which the treatment laser light is to be emitted is located (i.e., a rough direction in which the irradiation spot is located in the observation image using the contact lens 26). The outer circumferential indicator 75 is displayed along the outer circumference of the observation field of view by the operator via the observation optical system 40. As described above, the observation optical axis of the observation optical system 40 and the center of the display area of the display unit 50 are aligned with each other (i.e., match). Therefore, by displaying the outer circumferential indicator 75 along the outer circumference of the observation field with the center of the display area of the display unit 50 as a reference, the operator can easily recognize the appropriate direction.

Specifically, in this embodiment, the plurality of outer circumferential indicators 75 are arranged in an arc-shape or an annular-shape along the outer circumference of the observation field. Further, when moving one of the outer circumferential indicators 75, the control unit 60 moves it on an arc-shape or annular-shape line along the outer circumference of the observation field. The center of the arc-shape or annular-shape line on which the outer circumferential indicators 75 are displayed (in other words, the center of the outer circumferential indicators 75 or the center of curvature of the outer circumferential indicators 75) is aligned with the observation optical axis of the observation optical system 40. Therefore, the operator can appropriately recognize the direction indicated by the outer circumferential indicators 75.

The outer circumferential indicators 75 include a next direction indicator 75A. The next direction indicator 75A indicates a directional guide of the next irradiation spot to be irradiated with the treatment laser light among the plurality of irradiation spots defined by the irradiation plan. Each time the laser irradiation is performed, the control unit 60 shifts the position of the next direction guide 75A to a position corresponding to an irradiation spot that is defined by the irradiation plan and is located adjacent in a forward direction. For example, in the case of the irradiation plan in which 100 irradiations are performed in a clockwise direction over the entire circumference of the trabecular meshwork TM**,** the next direction guide 75A may be shifted by 3.6 degrees relative to the center O. Therefore, the operator can appropriately recognize a direction of the irradiation spot whose aiming point is to be adjusted next time by referring to the next direction indicator 75A that is shifted along the outer circumference of the observation field.

The outer circumferential indicators 75 further include a completed direction indicator 75B. The completed direction indicator 75B indicates a directional guide of the irradiation spot that has already been irradiated with the treatment laser light, among the plurality of irradiation spots defined by the irradiation plan. Each time the laser irradiation is completed, the control unit 60 replaces the next direction indicator 75A, which was displayed just before the irradiation, with the completed direction indicator 75B. Therefore, the operator can appropriately adjust the next aiming point using the next direction indicator 75A by recognizing the direction in which the laser irradiation has been completed. It also becomes easier to recognize the progress of treatment.

The outer circumferential indicator 75 further includes a pre-irradiation direction indicator 75C. The pre-irradiation direction indicator 75C indicates a directional guide of an irradiation spot that is scheduled to be irradiated with the treatment laser light subsequent to the next time, among the plurality of irradiation spots defined in the irradiation plan. Each time the laser irradiation is completed, the control unit 60 replaces the pre-irradiation direction indicator 75C displayed in the direction of the irradiation spot in the next irradiation order defined in the irradiation plan with the next direction indicator 75A. Therefore, transition of the next direction indicator 75A is performed. In this case, the operator can appropriately adjust the next aiming point using the next direction indicator 75A by recognizing the direction of the irradiation spot that is scheduled to be irradiated with the treatment laser light after the next time. It also becomes easier to recognize the progress of treatment.

Note that the control unit 60 displays each of the next direction indicator 75A, the completed direction indicator 75B, and the pre-irradiation direction indicator 75C in a distinguishable manner (that is, in a different displaying manner). Therefore, the operator can easily recognize the type of the outer circumferential indicators 75 being displayed. Note that the control unit 60 may selectively display one or two of the next direction indicator 75A, the completed direction indicator 75B, and the pre-irradiation direction indicator 75C.

The guide 90 serves as a marker for making a target position on the tissue at which treatment laser light is to be emitted match one of the plurality of irradiation spots at which the treatment laser light is planned to be emitted next time. The control unit 60 displays the guide 90 according to the predetermined irradiation plan.

The guide 90 in this embodiment also serves as a spot interval guide showing proper spacing between the multiple irradiation spots at which the treatment laser light is planned to be emitted. Thus, an operator can confirm the guide 90 while observing the observation image of the tissue of the patient's eye and adjust the position of the multiple irradiation spots on the tissue by referring to the appropriate intervals indicated by the guide 90. Therefore, the operator can easily cause the interval between the irradiation spots, to which treatment laser light is applied, to be an appropriate interval.

In the present embodiment, the control unit 60 aligns the intervals between the plurality of indicators 90A, 90B, 90C included in the guide 90 with the appropriate intervals between the plurality of irradiation spots. Thus, by emitting treatment laser light multiple times according to the intervals of the multiple indicators 90A, 90B, 90C in the guide 90, the operator can easily bring the spacings between the multiple irradiation spots close to the appropriate intervals. Therefore, it becomes easier to perform treatment in accordance with the treatment plan more appropriately.

The control unit 60 changes the intervals between the plurality of indicators of the guide 90 displayed on the display unit 50 according to the magnification (or zoom ratio) of the observation optical system 40. That is, the control unit 60 increases the intervals between the multiple indicators of the guide 90 as the magnification of the observation optical system 40 increases. Therefore, even if the observation magnification is changed, the guide 90 corresponding to the appropriate intervals between the plurality of irradiation spots can be displayed on the display unit 50.

The control unit 60 displays a next aiming indicator 90A in the guide 90 to adjust a next target position on the tissue to be irradiated with the treatment laser light next time. Each time the laser irradiation to one irradiation spot is executed, the control unit 60 shifts the position of the next aiming indicator 90A to an adjacent position that is shifted by one appropriate interval of the plurality of irradiation spots in the forward direction defined by the irradiation plan. Accordingly, the operator can more easily adjust the next aiming point of the treatment laser light by adjusting the target position on the tissue at which the treatment laser light will be irradiated next time to the displayed next irradiation indicator 90A.

As described in the present embodiment, when the multiple indicators arranged corresponding to the multiple irradiation spots are included in the guide, the indicators other than the next aiming indicator 90A are post-irradiation indicators 90B corresponding to the spots to which the treatment laser light has been emitted or pre-irradiation indicators 90C corresponding to the spots to which the treatment laser light has not been emitted. As shown in FIG. 10, the control unit 60 displays the next aiming indicator 90A in a different manner (that is, in a manner distinguishable by the operator) from the post-irradiation indicator 90B and the pre-irradiation indicator 90C. Thus, the operator can easily recognize the position of the next aiming indicator 90A. In detail, the control unit 60 displays each of the next aiming index 90A, the post-irradiation index 90B, and the pre-irradiation index 90C in mutually different manners. Therefore, the operator can easily grasp the positional relationship between the next aiming indicator 90A, the post-irradiation indicator 90B, and the pre-irradiation indicator 90C, making it easier to proceed with the treatment more smoothly.

The ophthalmic laser treatment device 1 in the present embodiment displays an end of each of the indicators in the guide 90 close to the aiming point for the laser light (in FIG. 10, a lower end of each of the indicators) to be aligned with a curve line following the curve of the treatment site of the patient's eye E (in the present embodiment, the trabecular meshwork TM) having an arc or annular shape. Thus, when the guide 90 is set at an appropriate position with an appropriate orientation with respect to the next treatment target site, distances between one ends (lower ends in FIG. 10) of the multiple indicators and the arc-shaped treatment target site are reduced. As a result, by referring to the guide 90, the operator can more appropriately adjust the next aiming point of the treatment laser light. In the example shown in FIG. 10, the guide 90 is displayed on a side opposite to the iris when viewed from the treatment target site (the trabecular meshwork TM in this embodiment). However, the guide may be displayed on the side of the iris rather than the treated area.

Note that although the shape of the curve of the treatment target site of the patient's eye E may be different from other patient's eyes, the treatment target site does not have a significant change in each patient's eye. Therefore, the aiming point side end of each of the plurality of indicators may be displayed along a predetermined curve that is determined based on the average shape of the treatment target sites. As one example, in this embodiment, an experiment has been performed in advance where a portion of the model eye that imitates the trabecular meshwork (a simulated trabecular meshwork) is observed under predetermined conditions. A program for displaying the aiming point side end of each of the plurality of indicators is stored in advance in a nonvolatile memory 65 in accordance with the shape of the simulated trabecular meshwork observed in the experiment.

The ophthalmic laser treatment device 1 in the first embodiment displays some elements of each indicator included in the guide 90 by arranging the elements along a straight line. In this embodiment, the ophthalmic laser treatment device 1 displays the indicators 90A, 90B, 90C included in the guide 90 along a virtual linear angle reference line AL (which is not actually displayed on the display unit 50). In this embodiment, the center of each of the plurality of indicators is displayed along the angle reference line AL. In this case, the operator can proceed with the treatment more appropriately by aligning the direction, in which the plurality of indicator elements are arranged, with the shifting direction in which the aiming point of the treatment laser light is shifted to the next irradiation spot. For example, the reflective surface of a contact lens may include an edge or the like perpendicular to a direction extending outward from the central axis of the lens. In this case, the operator aligns the direction of the edge of the contact lens reflected in the observed image with the linear direction in which multiple index elements are arranged, and aims the treatment laser light along the direction of the edge. It is also possible to move it. In addition, even if the contact lens that was in contact with the patient's eye is once removed from the patient's eye, the operator can orient the edge of the contact lens in the observed image to a straight line on which multiple index elements are lined up. It is also possible to easily restore the position and direction of the contact lens by adjusting the contact lens to the correct direction.

Specifically, in this embodiment, each of the plurality of indicators is perpendicular to the angle reference line AL, and the center (more specifically, the center of gravity) of each indicator is located on the angle reference line AL. Therefore, when the plurality of indicators of the guide 90 are arranged at appropriate positions with respect to the next irradiation spot, each indicator of the guide 90 extends from the angle reference line AL to a position close to the treatment target site (trabeculae). As a result, the positional relationship between the guide 90 and the treatment target site (for example, the positional relationship between a specific indicator and a characteristic portion in the tissue) can be more easily recognized. The length of each of the plurality of indicators of the guide 90 is symmetrical about the angle reference line AL. Therefore, the operator can easily recognize the direction in which the plurality of indicators are arranged (that is, the direction in which the angle reference line AL extends), so the operator can easily adjust the angle of the reflective surface of the contact lens to an appropriate angle. For example, by adjusting the angle of the reflective surface of the contact lens so that the angle reference line AL is in contact with the annular-shaped or arc-shaped treatment site, it is possible to adjust the angle of the reflective surface to an appropriate angle. Note that the multiple indicators of the guide 90 do not have to extend from the angle reference line AL to close to the treatment site. Further, the indicators displayed near the treatment target site may have appropriate widths to absorb individual differences in the curves of the treatment target sites.

Moreover, the ophthalmic laser treatment device 1 in the first embodiment causes the display unit 50 to display the total number of irradiation spots defined by the irradiation plan. In the example shown in FIG. 10, the denominator number of "SHOTS" is the total number of irradiation spots. Furthermore, the number of irradiation spots for which treatment has been completed (that is, the total number of irradiation spots for which irradiation with the treatment laser light has been completed and the number of irradiation spots for which irradiation has been skipped) is also displayed. In the example shown in FIG. 10, the molecule number of "SHOTS" is the number of irradiation spots for which treatment has been completed. Each time laser irradiation or irradiation skip is executed, "1" is added to the molecule number of "SHOTS". Further, in the example shown in FIG. 10, the mode of the aiming light being used and the energy of the treatment laser light are also displayed.

The flow of treatment using the guide 90 will be described using an example shown in FIG. 11. In FIG. 11, parts of the operator's observation field other than the area around the guide 90 displayed on the display unit 50 are omitted only for understanding the transition of the display of the guide 90 and the transition of the irradiation position of the treatment laser light and the aiming light (i.e., the irradiation position AI of the aiming light in FIG. 11). FIG. 11 shows one example with an irradiation plan set in advance by the operator where irradiation is performed in a clockwise direction step by step on an actual trabecular meshwork TM from the lower side to the diagonally lower left side. In FIG. 11, the lower trabecular meshwork TM is reflected by the reflective surface of the contact lens and enters into the operator's observation field. Accordingly, in FIG. 11, the top and bottom are reversed, and the lower trabecular TM is reflected (i.e., appears) in the operator's observation field of view. When the treatment for the irradiation spots proceeds from right to left (that is, a counterclockwise direction) on the reflective surface in FIG. 11, in the actual lower trabecular meshwork TM, the treatment for the multiple irradiation spots proceeds from right to left. (i.e., a clockwise direction).

As shown in FIG. 11 (a), the control unit 60 determines the angle of the guide 90 to be displayed on the display unit 50 according to the progress of the irradiation plan, and displays the guide 90 at the determined angle. The guide 90 shown in FIG. 11 is used for irradiating a lower area of the actual trabecular meshwork TM ten times (a specified number of times, that is, 10 times at most). The multiple indicators of the guide 90 shown in FIG. 11 (a) are arranged in a horizontal direction in order to treat the lower area of the trabecular meshwork TM. The control unit 60 causes the next aiming indicator 90A, to which the next treatment laser light will be emitted, to be identifiably displayed in the guide 90 In the example shown in FIG. 11 (a), the control unit 60 first displays the next aiming indicator 90A at a position of an end of the guide 90 (in FIG. 11 (a), the indicator located at the right end of the guide 90) opposite to the forward direction (the left direction in this embodiment) of treatment that is defined by the irradiation plan. Among the plurality of indicators of the guide 90, indicators other than the next aiming indicator 90A are the pre-irradiation indicators corresponding to at least some of the spots to which the treatment laser light will be emitted subsequent to the next time. Furthermore, when starting displaying the guide 90, the control unit 60 sets the irradiation position AI of the treatment laser light and the aiming light at a position indicated by the next aiming indicator 90A (in the present embodiment, a position close to one end of the next aiming indicator 90A in an extending direction) by controlling the irradiation position moving unit 29. In this embodiment, the irradiation position AI at the start timing of displaying the guide 90 is set at a position so as not to overlap with the next aiming indicator 90A. Accordingly, it is possible to avoid a situation where the irradiation position AI indicated by the aiming light cannot not be recognized by the operator due to the next aiming indicator 90A.

The operator adjusts at least one of a rotation angle of the contact lens or a relative position (i.e., "the relative position of the device") between the patient's eye E that is moved by the joystick 5 and the laser irradiation optical system 10 so that the next aiming indicator 90A in the guide 90 is aligned with the target position on the treatment target site of the patient's eye E (in the present embodiment, the trabecular meshwork TM). Note, in FIG. 11 (a) and FIG. 11 (b), the operator may adjust at least one of the rotation angle of the contact lens and the relative position of the device so that the irradiation position AI indicated by the aiming light matches the initial target position on the tissue. Furthermore, by adjusting at least one of the contact lenses and the relative position of the device, the operator moves a curved line formed by of one ends of the multiple indicators in the guide 90 that are close to the aiming spot of the treatment laser light to be close to the curve line of the treatment target site of the patient's eye E having an arc-shape or an annular shape. Here, if any characteristic site exists in the tissue of the patient's eye E included in the observation image, it would be preferable for the operator to recognize the positional relationship between at least one of the indicators of the guide 90 (i.e., a specific indicator) and the characteristic site of the tissue. In this case, in subsequent treatment procedures, the operator can restore the positional relationship between the guide 90 and the tissue to the original relationship by adjusting the positional relationship between the specific indicator and the characteristic part of the tissue to the same positional relationship even when the positional relationship between the guide 90 and the tissue of the patient's eye E is changed. In the state shown in FIG. 11 (b), the operator inputs an instruction for performing irradiation with treatment laser light into the ophthalmic laser treatment device 1. As a result, treatment laser light is emitted at the irradiation position AI which is indicated by the aiming light.

As shown in FIG. 11 (b), (c), each time one laser irradiation is executed, the control unit 60 shifts the irradiation position AI of the treatment laser light and the aiming light to an adjacent position that is shifted by one appropriate interval between the irradiation spots in the forward direction defined by the irradiation plan. Thus, the irradiation position AI of the treatment laser light and the aiming light automatically moves to, or near, the target position at which the treatment laser light will be emitted next time (i.e., the position on the tissue where the irradiation spot is scheduled to be emitted next time) each time the treatment laser light is emitted without changing the relative position of the device and the angle of the contact lens by the operator.

In this embodiment, when an instruction for moving the irradiation position AI of the treatment laser light and the aiming light are input via the irradiation position operation unit 9, the control unit 60 moves the irradiation position AI on the tissue in response to receiving the input instruction. Thus, the operator can move the irradiation position AI to a desired position only by inputting instructions for moving the irradiation position AI into the ophthalmic laser treatment device 1 without changing the relative position of the device and the angle of the contact lens. Thus, it is easy to adjust the aiming point of the treatment laser light with a reduced amount of movement of the observation image. Note, since the shape of the treatment target site varies, the irradiation position AI may deviate from the target position on the tissue at which the treatment laser light will be emitted next time, after the irradiation position AI automatically moved based on the irradiation plan. However, by inputting instructions for moving the irradiation position AI into the ophthalmic laser treatment device 1, the operator can also slightly adjust the irradiation position AI to the target position while the amount of movement of the observation image is reduced. Thus, the accuracy and efficiency of treatment can be further improved. Note, even if the irradiation position AI that has moved automatically deviates from the target position on the tissue at which the treatment laser light will be emitted next time, the operator only needs to slightly adjust the irradiation position AI in a direction perpendicular to the extending direction of the trabecular meshwork. In this case, since the movement by one spot has already been performed automatically, the operator only needs to slightly move the irradiation position AI by, e.g., rotating the contact lens.

Further, one irradiation by the treatment laser light is completed, the control unit 60 shifts the position of the next aiming indicator 90A to an adjacent position that is shifted by one appropriate interval of the plurality of irradiation spots in the forward direction determined by the irradiation plan. Accordingly, the operator can more easily adjust the next aiming point by shifting the target position on the tissue at which the treatment laser light will be irradiated next time to the displayed next irradiation indicator 90A. In this embodiment, the next irradiation position AI is automatically shifted by one spot each time treatment laser light is emitted. Thus, while enjoying simplifying operation during adjacent irradiation by the automatic shifting, the operator can determine whether to perform irradiation or skip (non-irradiate) while observing the state of the trabecular meshwork (for example, the pattern of the trabecular meshwork) for each spot. Also, since the operator only needs to set a point (one spot) onto the trabecular meshwork (a line), there is no need for complicated aligning operation to set the line (an arc pattern) along the line (the trabecular meshwork extending), such as performing an arc pattern irradiation with multiple irradiation spots arranged in an arc shape. Note, when the arc pattern is aligned to the trabecular meshwork, it is more difficult to align the arc pattern with the trabecular meshwork in proportion to the length of the arc pattern.

In the state shown in FIG. 11 (c), the operator inputs an instruction for performing irradiation with treatment laser light into the ophthalmic laser treatment device 1. As a result, treatment laser light is emitted at the irradiation position AI which is indicated by the aiming light. Thereafter, treatment laser light is emitted ten times, which is a predetermined number of emission times (that is, ten times at most) on one irradiation section in the trabecular meshwork TM by the procedure similar to the procedure described with reference to FIG. 11 (b) and FIG. 11 (c)) (see FIG. 11 (c) to FIG. 11 (k)). The irradiation section is an angular range with an arc-shape where treatment laser light irradiation is scheduled to be performed a specified number of M times (M ≥ 2, more specifically, M = 10 in the example shown in FIG. 11) while the display position and angle of the guide 90 on the display unit 50 are fixed.

As shown in FIGS. 11 (k) and FIG. 11 (l), the control unit 60 rotates the entire guide 90 in the forward direction by the angle of one irradiation section each time laser light irradiation onto all the plurality of irradiation spots (ten spots in FIG. 11) in the irradiation section under treatment is completed. In other words, when treatment for one irradiation section is completed while the display position and angle of the guide 90 are fixed, the displayed guide 90 is rotated to have an angle corresponding to the next irradiation section. Accordingly, irradiation with the treatment laser light onto each of the plurality of irradiation sections is automatically and appropriately assisted according to the progress of the irradiation plan.

The angle of the irradiation section can be selected as appropriate. For example, the ophthalmic treatment device 1 according to the present embodiment divides the range of R degree (R<=360) in a ring-shaped or arc-shaped treatment target site (in the present embodiment, trabecular meshwork TM) into S irradiation sections. Then, the ophthalmic treatment device 1 adjusts the aiming point within each irradiation section. The control unit 60 rotates the entire guide 90 in the forward direction by an angle of one irradiation section (i.e., R/S degree) each time laser light irradiation onto all the plurality of irradiation spots in the irradiation section under treatment is completed. As a result, the laser irradiation is appropriately performed in each of the plurality of irradiation sections.

As shown in FIG. 11 (k) and FIG. 11 (l), after rotating the guide 90 for the next irradiation section, the control unit 60 sets the position of the next aiming indicator 90A at one end of the rotated guide 90 opposite to the forward direction of treatment that is defined by the irradiation plan. As a result, the position of the next aiming indicator 90A is appropriately changed according to the progress of the irradiation plan, making it easier for the treatment to proceed more smoothly. Furthermore, each time the guide 90 having the multiple indicators is rotated for the next irradiation section, the control unit 60 sets all the indicators except the next aiming indicator 90A as the pre-irradiation indicators 90C (see FIG. 10).

As shown in FIG. 11 (k) and FIG. 11 (l), after rotating the guide 90 for the next irradiation section, the control unit 60 moves, by controlling the irradiation position moving unit 29, the irradiation position AI of the treatment laser light and the aiming light in a direction opposite to the forward direction of treatment that is defined by the irradiation plan. As one example, the control unit 60 sets, after rotating the guide 90 for the next irradiation section, the irradiation position AI of the treatment laser light and the aiming light at a position indicated by the next aiming indicator 90A (in the present embodiment, a position close to one end of the next aiming indicator 90A in an extending direction of the indicator 90A) by controlling the irradiation position moving unit 29. As a result, treatment for the next irradiation section can be started smoothly and easily.

In FIG. 11 (l) and FIG. 11 (m), the operator adjusts at least one of the rotation angle of the contact lens and the relative position of the device so that the position of the next aiming indicator 90A included in the guide 90 is aligned with (i.e., matches) the next target position on the tissue of the patient's eye E. Note, in FIG. 11 (l) and FIG. 11 (m), the operator may adjust at least one of the contact lens and the relative position of the device so that the irradiation position AI indicated by the aiming light matches the next target position on the tissue. Furthermore, by adjusting at least one of the rotation angle of the contact lens and the relative position of the device, the operator moves a virtual curved line formed by of one ends of the multiple indicators in the guide 90 that are close to the aiming spot of the treatment laser light to be aligned with the curve line of the treatment target site of the patient's eye E having an arc-shape or an annular shape. At the state shown in FIG. 11 (m), the operator inputs the irradiation execution instruction into the ophthalmic laser treatment device 1 to irradiate the target position with treatment laser light. Thereafter, the treatment proceeds similar to FIG. 11 (k) to FIG. 11 (k).

In the present disclosure, a position that deviates from the irradiation spot at which irradiation with treatment laser light was completed the last time in the forward direction defined by the irradiation plan by "n" appropriate intervals of the irradiation spots (n=1 in this embodiment) is defined as an adjacent position. As described above, in FIG. 11 (b) to FIG. 11 (k), the multiple irradiation spots are irradiated with the treatment laser light while the position and angle of the guide 90 are fixed. Accordingly, in FIG. 11 (b) to FIG. 11 (k), the next aiming point indicated by the next aiming indicator 90A is the adjacent position. On the other hand, the range within which the operator can observe the observation image by the observation optical system 40 and the range in which the irradiation position AI of the treatment laser light and the target light can be moved by the irradiation position moving unit 29 are both limited. Thus, when the arrangement of the multiple irradiation spots on the tissue is adjusted by the guide 90 that is superimposed on the observation image while the observation image is fixed, the next aiming point indicated by the guide 90 may deviate from the observation image, or the irradiation position AI of the light may deviate from the movable range. Therefore, in order to keep the next aiming point 90A indicated by the guide 90 within the observation image and keep the light irradiation position AI within the movable range, it is also necessary for the control unit 60 to move the next aiming point to a non-adjacent position different from the adjacent position. As shown in FIG. 11 (k) and FIG. 11 (l), in this embodiment, the control unit 60 may move the next aiming point to the non-adjacent position different from the adjacent position PP shown in FIG. 11 (k) (the position indicated by the next aiming indicator 90A in FIG. 11 (l)). In this case, by adjusting at least one of the rotation angle of the contact lens and the relative position of the device so that the position indicated by the next aiming indicator 90A included in the guide 90 corresponds to the next target position on the tissue of the patient eye E, the operator can adjust the arrangement of the irradiation spots again while the range of the observation image is fixed.

However, if the next aiming point is moved to the non-adjacent position different from the adjacent position PP, the operator would lose sight of the target position on the tissue appearing in the observation image (the position at which the treatment laser light will be emitted next time). On the contrary, when the next aiming point indicated by the guide 90 is moved to the non-adjacent position different from the adjacent position PP (see FIG. 11 (k)), the ophthalmic laser treatment device 1 according to the present embodiment causes the display unit 50 to display the target position indicator 92 indicating a position on the tissue in the adjacent position PP (that is, a position would be indicated by the next aiming indicator 90A if the next aiming indicator 90A was moved to the adjacent position). The position on the tissue indicated by the target position indicator 92 becomes a target position to which the next aiming point of the guide 90 is aligned. Thus, the operator can appropriately adjust the aiming point of the treatment laser light to the target position by recognizing the target position on the tissue by the target position indicator 92 interposed on the observation image (for example, memorize characteristics of the pattern of the trabecular meshwork indicated by the target position indicator 92) and by aligning the recognized target position with the position indicated by the next aiming indicator 90A. Accordingly, it is possible to appropriately support treatment with treatment laser light when a contact lens having a reflective surface is used.

As described above, the control unit 60 according to the present embodiment rotates the entire guide 90 in the forward direction by an angle of one irradiation section each time laser light irradiation to all the plurality of irradiation spots within the current irradiation section under treatment is completed. When the entire guide 90 is rotated, the control unit 60 causes the display unit 50 to display the target position indicator 92 indicating the target position on the tissue in the adjacent position PP (that is, the organizational position that would be indicated by the next aiming indicator if the next aiming indicator moved to the adjacent position). Accordingly, irradiation with the treatment laser light to each of the plurality of irradiation sections is automatically and appropriately assisted according to the progress of the irradiation plan.

As shown in FIG. 11 (l), the target position indicator 92 displayed on the display unit 50 in this embodiment is a mark surrounding the target position which was the adjacent position PP at the time of displaying the target position indicator 92 (that is, the position on the tissue which would be indicated by the next aiming indicator 90A if the next aiming indicator 90A was moved to the adjacent position PP). Thus, the operator can recognize the state of the tissue at the target position located inside the mark using the observation image on which the mark is superimposed. Thus, it is easier for the operator to appropriately set the tissue located inside the mark at the position indicated by the next aiming indicator 90A.

Note, in the example shown in FIG. 11 (l), an annular mark surrounding the target position (i.e., the adjacent position PP at the time of start of displaying the target position indicator 92) is used as the target position indicator 92. However, the mark surrounding the target position is one example and other types of marks may be used. For example, marks, etc., arranged at multiple locations to surround the target position (for example, at four corners of a rectangular region surrounding the target position) may be used as the target position indicator.

However, it is also possible to change the displaying manner of the target position indicator. For example, the target position indicator may be a mark superimposed on the adjacent position PP (i.e., the target position). Even in this case, the operator can appropriately recognize the target position PP based on the state or the like of the tissue around the mark superimposed on the adjacent position PP (i.e., the target position). Also, the target position indicator may indicate the target position by indicating a position associated with the adjacent position PP (i.e., the target position). For example, the control unit 60 may indicate the target position by indicating, using the target position indicator, the position of the irradiation spot that was irradiated with treatment laser light just before. In this embodiment, the adjacent position PP (the target position) is a position that deviates from the position irradiated with treatment laser light just before. The adjacent position PP deviates in the forward direction of the treatment defined by the irradiation plan by one appropriate interval between the irradiation spots. Thus, the operator can properly recognize the target position (the adjacent position PP adjacent to the irradiation spot where the treatment laser light was irradiated immediately before) by recognizing, using the target position indicator, the position of the irradiation spot where the treatment laser light was emitted immediately before.

As shown in FIG. 11 (m) and FIG. 11 (n), the control unit 60 displays the target position indicator 92 on the display unit 50, and then removes the displayed target position indicator 92 upon receiving an instruction for executing treatment laser light irradiation. FIG. 11 (l) and FIG. 11 (m) show a situation where the position of the next aiming indicator 90A indicated by the guide 90 is moved to a position different from the adjacent position PP (see FIG. 11 (k)). At this time, the operator recognizes the target position on the tissue that will be irradiated with treatment laser light next time using the displayed target position indicator 92. Then, the operator sets the recognized target position on the tissue at the position of the next target indicator 90A, and inputs the irradiation execution instruction into the ophthalmic laser treatment device 1. Therefore, after the irradiation execution instruction is input, there is no need to display the target position indicator 92 since the target position on the tissue has been already set on the next aiming indicator 90A. Thus, the control unit 60 can remove the target position indicator 92 at an appropriate timing by inputting the irradiation execution instruction.

As shown in FIG. 11 (l) and FIG. 11 (n), the control unit 60 of the first embodiment causes the position at which the target position indicator 92 is superimposed and displayed by the display unit 50 to follow the target position on the observation image as the observation image by the observation optical system 40 moves. Thus, even if the target position on the tissue indicated by the target position indicator 92 is not memorized, the operator can easily and appropriately set the target position at the position indicated by the next aiming indicator 90A by considering the target position 92 that is moving to follow the target position. Note that a specific method for causing the superimposed position of the target position indicator 92 to follow the target position on the observation image can be selected as appropriate. In the first embodiment, the following method is selected. The control unit 60 specifies the target position on the observation image that is moving. The target position is specified (learning image characteristics by setting the region on the observation image on which the above-described target position indicator 92 is interposed as an ROI region) by using known image processing (e.g., pattern matching) on the observation images that are continuously captured by the imaging unit (see FIG. 2). Then, the control unit 60 moves the target position indicator 92 to follow the specified target position.

Note that the ophthalmic laser treatment device 1 in the present embodiment is configured to receive an instruction for rotating the entire guide 90 from a user before completing irradiation to all the irradiation spots within the current irradiation section (that is, during the treatment on the irradiation spots in the irradiation section). If an instruction to rotate the entire guide 90 is input before the treatment for all the irradiation spots in the current irradiation section is completed, the ophthalmic laser treatment device 1 rotates the entire guide 90 by an angle corresponding to an area of the current irradiation section where the treatment has been completed. Therefore, even if the treatment for each irradiation section is not completed, the operator can move forward treatment for the next irradiation section by rotating the entire guide 90.

Here, the angle between the two adjacent irradiation spots is A degrees when viewed from the center of a circle through which multiple planned irradiation spots pass, and the number of the irradiation spots for which treatment has been done within the irradiation section under treatment is m. In this embodiment, the total angle corresponding to the area where the treatment has been done in the current irradiation section is calculated by "A (degrees) x m (number)". The number of irradiation spots "m" for which treatment has been done also includes the number of irradiation spots for which irradiation with the treatment laser light was skipped in accordance with a skip instruction, which will be described later.

Further, the ophthalmic laser treatment device 1 in the present embodiment is configured to receive an instruction (hereinafter referred to as a "skip instruction") from a user to omit irradiation with the treatment laser light to the next irradiation spot defined in the irradiation plan. When the skip instruction is input, the control unit 60 moves the irradiation position AI of the treatment laser light and the aiming light to the adjacent position without emitting the treatment laser light. Also, when the skip instruction is input, the control unit 60 moves the position of the next aiming indicator 90A to the adjacent position deviated by "n" appropriate intervals (n=1 in this embodiment) of the irradiation spots in the forward direction defined by the irradiation plan without emitting the treatment laser light. Therefore, if a non-irradiation area where irradiation with the treatment laser light should not be performed, the operator inputs the skip instruction to skip the irradiation with the treatment laser light for the non-irradiation area. Thus, the operator restarts treatment from the irradiation spot that is planned to be irradiated next. Therefore, it becomes easier for the treatment to proceed more smoothly. That is, the ophthalmic laser treatment device 1 includes a skip means that is configured to skip the step of irradiating, with the treatment laser light, at least one of the plurality of irradiation spots defined in the treatment plan. Therefore, both convenience provided from the treatment plan (for example, guiding the irradiation with treatment laser light) and flexible treatment realized by the skip means (for example, a step of skipping irradiation with the laser light for the non-irradiated area that was found after starting the treatment in accordance with the treatment plan) can be obtained.

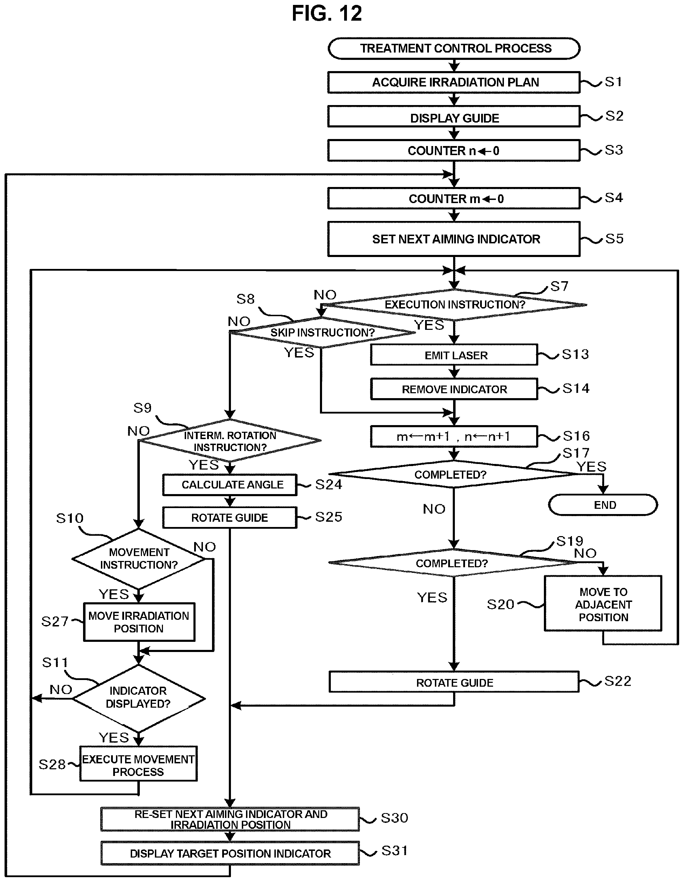

With reference to FIG. 12, a treatment control process in the first embodiment will be described. Note that all treatment control processing described below is executed by the CPU (controller) 61 of the control unit 60 when an instruction for starting treatment is input via a touch panel or the like. The CPU 61 executes the treatment control process according to a control program stored in the ROM 62 or nonvolatile memory 65.

First, the control unit 60 obtains the irradiation plan on treatment laser light for the patient's eye E (S1). As described above, when the patient's eye E is irradiated with the treatment laser light using the contact lens 26 having the reflective surface 27, the irradiation order etc. for multiple irradiation spots scheduled to be irradiated with the treatment laser light are defined in the irradiation plan. In the irradiation plan according to the present embodiment, the irradiation range of treatment laser light (i.e., a circumferential range where the plurality of irradiation spots are arranged) at an annular treatment target site (trabecular meshwork TM in this embodiment), the irradiation spot at which the treatment laser light should be emitted first, the angle between two adjacent irradiation spots, the angle of one irradiation section, the number M of times treatment laser light is emitted within each irradiation section, the total number N of times treatment laser light is emitted over the entire treatment target site, the irradiation order (including the irradiation direction) of treatment laser light are defined. Note that when the angle range of the annular-shaped or arc-shaped treatment target site is R degrees (R<=360) and the number of irradiation spots on which irradiation with the laser light in the treatment target site is performed is N, then the angle between two adjacent irradiation spots can be defined as "R/N" degrees. Furthermore, when dividing the range of R degrees in a ring-shaped or arc-shaped treatment target site into S irradiation sections, the angle of one irradiation section is defined as "R/S" degrees. In this embodiment, each time one irradiation with the treatment laser light is performed, the irradiation spot adjacent to the irradiation spot for which the irradiation has been completed turns to be the next irradiation spot that will be irradiated with the treatment laser light next time. The multiple irradiations with the treatment laser light are sequentially performed in a clockwise or counterclockwise direction. As an example, in this embodiment, the irradiation plan is prepared by operating the control box 6.

The control unit 60 controls the display unit 50 to display the guide 90 at an angle corresponding to the orientation of the first irradiation section (S2). The control unit 60 sets the value of the total irradiation number counter "n", which specifies the cumulative number of times the treatment laser light is emitted to the entire treatment target site, to "0" (S83). As a result, the observation field of view by the operator is shown as an example in FIG. 11 (a).

The control unit 60 sets, to "0", the value of the entire irradiation number counter "n" that specifies the number of irradiations of the treatment laser light to all the plurality of irradiation spots defined in the irradiation plan (S3). Furthermore, the control unit 60 sets the value of the single-section irradiation number counter "m" that specifies the number of times the treatment laser light is irradiated within one irradiation section to "0" (S4). The control unit 60 sets a displaying position of the next aiming indicator 90A, which is the indicator for next irradiation with the treatment laser light, among the plurality of indicators in the guide 90, so that the next aiming indicator 90A is overlapped with the indicator located at one end of the guide 90 opposite to the forward direction in the irradiation order (S5).

The control unit 60 determines whether the operator has input an instruction for performing irradiation with the treatment laser light (S7). If the irradiation execution instruction is not input (S7: NO), the control unit 60 determines whether an irradiation skip instruction (that is, the "skip instruction") for skipping irradiation to the next irradiation spot defined by the irradiation plan (S8). If the skip instruction is not input (S8: NO), the control unit 60 determines whether an instruction for rotating the entire guide 90 before irradiation to all the multiple irradiation spots in the current irradiation section is completed (hereinafter, referred to as "intermediate rotation instruction") has been input (S9). If the intermediate rotation instruction is not input (S9:NO), the control unit 60 determines whether an instruction for moving the irradiation position AI of the treatment laser light and the aiming light by the irradiation position moving unit 29 is input (S10). If the instruction for move the irradiation position is not input (S10: NO), the control unit 60 determines whether the target position indicator 92 (see FIG. 11 (l) and FIG. 11 (m)) is displayed on the display unit 50 (S11). If no instructions are input at S7 to S11, the steps at S7 to S11 are repeated to be a standby state.

When the instruction to perform laser irradiation is input (S7: YES), the treatment laser light is emitted (S13). When the target position indicator 92 (see FIG. 11 (l) and FIG. 11 (m)) is displayed on the display unit 50, the control unit 60 removes the target position indicator 92 from the display unit 50 (S14) when the irradiation execution instruction is input. Next, the control unit 60 adds "1" to each of the total irradiation number counter "n" and the single-section irradiation number counter "m" (S16). The control unit 60 determines whether the value of the total irradiation number counter "n" has reached the total number "N" the treatment laser light is emitted to the entire treatment target site (S17). If the specified number of irradiations with the treatment laser light to all the irradiation spots (including the number of skipped irradiations in this embodiment) has not been completed (in other words, if "n" has not reached "N") ) (S17: NO), the control unit 60 determines whether the specified number of irradiations with the treatment laser light to the current irradiation section has been completed (that is, if the value of the single-section irradiation number counter "m" has reached the irradiation number "M" for the single irradiation section) (S19). If the treatment laser light irradiation to the current irradiation section has not been completed (S19: NO), the control unit 60 moves the irradiation position AI of the treatment laser light and the target light to the adjacent position that is offset by n appropriate intervals of the irradiation spots (n=1 in this embodiment) in the forward direction of treatment defined by the irradiation plan. Further, the control unit 60 shifts the position of the next aiming indicator 90A to an adjacent position that is shifted by n appropriate intervals of the irradiation spots (one interval in the present embodiment) in the forward direction defined by the irradiation plan (S20). Note, at S20, control is also performed to change the previous next aiming indicator 90A to the post-irradiation indicator 90B. The process then returns to S47.

When irradiation with the treatment laser light to the current irradiation section is completed (S19: YES), the control unit 60 rotates the guide 90 by the angle for the single irradiation section (i.e., the predetermined angle) (S22). The control unit 60 re-sets the next aiming indicator 90A and the pre-irradiation indicator 90C (S30). Furthermore, the control unit 60 displays the target position indicator 92 at the adjacent position PP (the position deviating from the irradiation spot at which irradiation of the treatment laser light was completed last time by n appropriate intervals of the irradiation spots in the forward direction of treatment defined by the irradiation plan) (S31). Then, the process returns back to S4. Thereafter, treatment on the next irradiation section is performed. When irradiation to all the irradiation spots with the treatment laser light is completed (S17: YES), the process ends.

Further, if the skip instruction is input before the instruction to execute laser irradiation is input (S8: YES), the control unit 60 adds "1" to each of the total irradiation number counter "n" and the single irradiation number counter "m" (S16), and executes the processes at S17, S19, S20, S22, S30, and S31.

Furthermore, if the intermediate rotation instruction is input before an instruction to execute irradiation with the treatment laser light is input (S9: YES), the control unit 60 calculates an angle corresponding to an area in the current irradiation section where treatment has been done as an angle at which the guide 90 should be rotated (S24). As described above, an angle between the two adjacent irradiation spots when viewed from the center of a virtual circle through which multiple planned irradiation spots pass is defined as A degrees, and the number of the irradiation spots in the current irradiation section where the treatment has been done is defined as "m" (in the present embodiment, the number m is equal to the single section irradiation number counter m). Then, in this embodiment, the control unit 60 calculates the total angle corresponding to the area where the treatment has been done in the current irradiation section by "A (degrees) x m (number)". The number of irradiation spots "m" for which treatment has been done also includes the number of irradiation spots for which irradiation with the treatment laser light was skipped in accordance with the skip instruction. The control unit 60 rotates the entire guide 90 by the angle calculated at S24 in the forward direction defined by the treatment plan (S25). Note that when the number "m" of the irradiation spots where treatment has been done is "0," the angle calculated by S24 becomes "0 degrees," so the guide 90 is not rotated at S25. Thereafter, re-setting the next aiming indicator 90A and the pre-irradiation indicator is performed (S30), then the target position indicator 92 is displayed on the adjacent position PP (S31), and the process returns to S4.

Also, when an instruction for moving the irradiation position AI of the treatment laser light and the aiming light by the irradiation position moving unit 29 is input before the irradiation execution instruction is input (S10: YES), the control unit 60 controls the irradiation position moving unit 29 to move the irradiation position AI of the treatment laser light and the aiming light to a position according to the input instruction. The process proceeds to S11. Thus, the operator can adjust the irradiation position AI without moving the observation image by just inputting the instruction for moving the irradiation position AI even when the irradiation position AI that was automatically moved at S20 deviates from the next target position on the tissue, for example.

Furthermore, when the target position indicator 92 (see FIG. 11 (l) and FIG. 11 (m)) is displayed on the display unit 50 (S11: YES), the control unit 60 of the first embodiment causes the position at which the target position indicator 92 is superimposed and displayed by the display unit 50 to follow the target position on the observation image, as the observation image by the observation optical system 40 moves (S28). The process then returns to S47. Since an example of the step of S28 has already been described, explanation is omitted here.

### <Second embodiment>

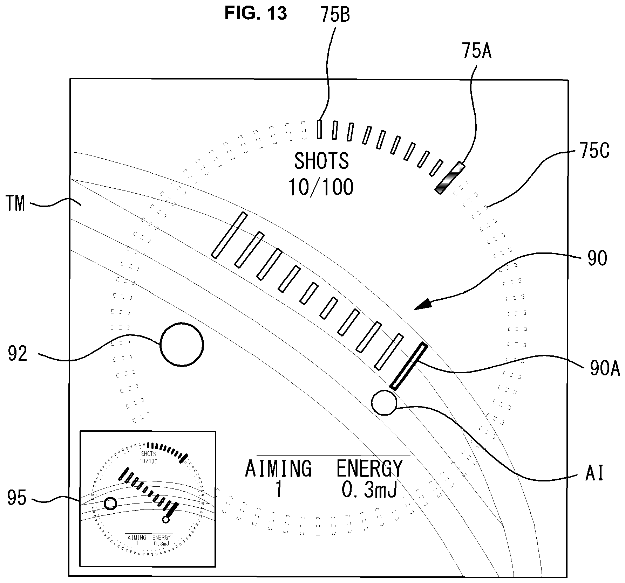

A process executed by an ophthalmic laser treatment device 1 according to a second embodiment will be described with reference to FIG. 13. Note that processing in the first embodiment can be applied partially or fully to the second embodiment. Accordingly, among the processing in the second embodiment, explanations are omitted or simplified for the content where processing in the first embodiment can be adopted.

In the first embodiment, the position at which the target position indicator 92 is superimposed and displayed by the display unit 50 is caused to follow the target position on the observation image, as the observation image by the observation optical system 40 moves. On the contrary, as shown in FIG. 13, the control unit 60 according to the second embodiment fixes the position of the target position indicator 92 displayed on the display unit 50. Even in this case, the operator can appropriately adjust the aiming point to the target position by aligning the target position on the tissue recognized by the target position indicator 92 with the position indicated by the next aiming indicator 90A.

As described above, the imaging unit 55 can capture an observation image on which an image is superimposed by the display unit 50. In the second embodiment, the control unit 60 presents a reference image 95 captured by the imaging unit 55 to an operator when the target position indicator 92 is displayed on the display unit 50 (for example, at the time of start of displaying the target position indicator 92). In this case, the operator recognizes again the target position on the tissue indicated by the target position indicator 92 by checking the provided reference image 95 even after the operator changed at least one of the relative position of the device and the angle of the contact lens to align the target position on the tissue with the position of the next aiming indicator 90A indicated by the guide 90 (that is, even when the actual target position on the tissue is offset from the position indicated by the target position indicator 92 that is fixedly displayed). Thus, the operator can appropriately adjust the target position on the tissue that is recognized by the target position indicator 92 to the position indicated by the next aiming indicator 90A.

In the example shown in FIG. 13, the control unit 60 displays the reference image 95 captured by the imaging unit 55 in a part of the display area of the display unit 50 where an image is superimposed on the observation image. Thus, by comparing the observation image of the tissue at this moment with the reference image 95 at the time of displaying the target position indicator 92, the operator can easily align the target position with the position indicated by the next aiming indicator 90A.

After presenting the reference image 95 to the operator, the control unit 60 stops presenting the reference image 95 when a irradiation execution instruction is input. As described above, after the irradiation execution instruction is input, there is no need to present the reference image 95 since the target position on the tissue has been already set on the next aiming indicator 90A. Thus, the control unit 60 can stop presenting the reference image 95 at an appropriate timing by inputting the irradiation execution instruction.

The embodiments disclosed herein are illustrative in all respects and should not be considered restrictive. The scope of the present invention is indicated by the claims rather than the above description, and it is intended that all changes within the meaning and scope of the claims and equivalents thereto are included. For example, it is also possible to adopt only some of the techniques described in each of the embodiments. Further, it is also possible to appropriately combine the techniques of a plurality of different embodiments and employ them in an ophthalmic laser treatment device 1.
he ophthalmic laser treatment device 1 in the above embodiment performs both the process for automatically moving the irradiation position of the treatment laser light and the aiming light based on an irradiation plan and the process for moving the irradiation position in response to receiving input instructions. However, the ophthalmic laser treatment device 1 may perform only one of the process for automatically moving the irradiation position based on the irradiation plan and the process for moving the irradiation position in response to receiving input instructions. For example, when the irradiation position automatically moved based on the irradiation plan deviates from the position at which the treatment laser light is scheduled to be emitted next time, the operator can adjust the irradiation position by slightly changing at least one of the relative position of the device and the angle of the contact lens. Even in this case, the amount of movement of the observation image is small.

The ophthalmic laser treatment device 1 in the above embodiment irradiates one irradiation spot with one shot of treatment laser light each time one irradiation execution instruction is input. It is assumed that a plurality of irradiation spots may be irradiated with treatment laser light each time one irradiation execution instruction is inputted. In this case, although the operator's workload is reduced and easier, the operator needs to accurately adjust all the multiple irradiation spots to the treatment target area and then input an irradiation execution instruction. On the other hand, when one shot of treatment laser light is emitted each time one irradiation execution instruction is inputted, the operator can adjust the target position of the treatment laser light for each of the multiple irradiation spots and then input the irradiation execution instruction. Thus, the position of each of the plurality of irradiation spots can be more accurately adjusted to the treatment target area. Further, it is easy to skip irradiation of non-irradiated areas with treatment laser light.

However, each time one irradiation execution instruction is input, the control unit 60 may control the irradiation position moving unit 29 based on the irradiation plan and emit treatment laser light multiple times at appropriate intervals to the multiple irradiation spots. In this case, the operator's workload is further reduced. For example, when irradiation is performed two times (for two spots) in response to receiving one irradiation execution instruction, the amount of work can be reduced with a similar operation feeling to the one irradiation (for one spot).

Further, useful effects can be obtained even if the next aiming indicator 90A is not displayed in a manner different from other indicators in the guide 90. For example, the control unit 60 may display multiple indicators on the display unit 50 at predetermined intervals (e.g., the appropriate interval between the multiple irradiation spots, etc.) without changing the displaying manner. Even in this case, the operator can appropriately adjust each of the multiple irradiation spots on an appropriate position on the tissue by referring to the multiple indicators.

## Claims

1. An ophthalmic laser treatment device that irradiates a tissue of a patient's eye with treatment laser light in response to receiving an irradiation execution instruction, comprising:
a treatment laser light source that is configured to emit treatment laser light;
an aiming light source that is configured to emit aiming light for an operator to recognize a scheduled irradiation position for the treatment laser light on the tissue;
an irradiation position moving unit that is configured to move, on the tissue, an irradiation position at which the treatment laser light and the aiming light are emitted;
an observation optical system that is configured to present an observation image of the tissue to the operator;
a display unit that is configured to display an image by superimposing the image on the observation image observed by the operator; and
a control unit, wherein
the control unit is configured to execute:
an irradiation plan acquisition step of acquiring an irradiation plan that defines an irradiation order of irradiations with the treatment laser light to a plurality of irradiation spots at which the treatment laser light is scheduled to be emitted when irradiating the patient's eye with the treatment laser light using a contact lens having a reflective surface that reflects the treatment laser light and the aiming light in a direction intersecting an optical axis;
a guide display step of controlling the display unit to display a guide in accordance with a progress of the irradiation plan, wherein the guide is used to align a target position on the tissue with a next irradiation spot of the plurality of irradiation spots at which the treatment laser light is scheduled to be emitted next time; and
an irradiation position movement step of moving the irradiation position of the treatment laser light and the aiming light by controlling the irradiation position moving unit.

2. The ophthalmic laser treatment device according to claim 1, wherein
the control unit is further configured to move, at the irradiation position movement step, the irradiation position of the treatment laser light and the aiming light to an adjacent position that is offset by n intervals of the plurality of irradiation spots in a forward direction defined by the irradiation plan each time irradiation with the treatment laser light to n irradiation spots of the plurality of irradiation spots are performed.

3. The ophthalmic laser treatment device according to claim 2, wherein
the control unit is further configured to move the irradiation position of the treatment laser light and the aiming light to the adjacent position without performing irradiation with the treatment laser light upon receiving an instruction for skipping irradiation with the treatment laser light to next n irradiation spots of the plurality of irradiation spots that are defined by the irradiation plan.

4. The ophthalmic laser treatment device according to any one of claims 1 to 3, wherein
when receiving an instruction for moving the irradiation position of the treatment laser light and the aiming light, the control unit is further configured to move, at the irradiation position movement step, the irradiation position in accordance with the instruction.

5. The ophthalmic laser treatment device according to any one of claims 1 to 4, wherein
the control unit is further configured to control, at the guide display step, the display unit to display a spot interval guide in accordance with a progress of the irradiation plan, and
the spot interval guide indicates intervals of the plurality of irradiation spots.

6. The ophthalmic laser treatment device according to claim 5, wherein
the spot interval guide includes a plurality of interval indicators, and
the control unit is further configured to align at least one of the plurality of interval indicators with the intervals of the plurality of irradiation spots.

7. The ophthalmic laser treatment device according to claim 5 or 6, wherein
the control unit is further configured to control the display unit to change a size of the spot interval guide in accordance with magnification of the observation image presented by the observation optical system.

8. The ophthalmic laser treatment device according to any one of claims 1 to 7, wherein
the control unit is further configured to execute:
display a next aiming indicator in the guide to set the target position on the tissue at which the treatment laser light is scheduled to be emitted next time; and
move the next aiming indicator to an adjacent position that is offset by n intervals of the plurality of irradiation spots in a forward direction defined by the irradiation plan each time irradiation with the treatment laser light to n irradiation spots of the plurality of irradiation spots is completed.

9. The ophthalmic laser treatment device according to any one of claims 1 to 8, wherein
the tissue of the patient's eye has an arc-shape or an annular shape,
the tissue is divided into a plurality of irradiation sections each having an arc-shape,
irradiations with the treatment laser light a predetermined number M (M>=2) of times are scheduled to be performed to each of the plurality of irradiation sections while a displayed position and an angle of the guide on the display unit are fixed, and
the control unit is further configured to rotate the entire guide by an angle corresponding to one irradiation section in a forward direction defined by the irradiation plan each time irradiation with the treatment laser light to all the plurality of irradiation spots in a currently-treated section of the plurality of irradiation sections is completed.

10. The ophthalmic laser treatment device according to claim 9, wherein
the control unit is further configured to rotate the entire guide by an angle corresponding to a partial area in a currently-treated irradiation section of the plurality of irradiation sections where treatment has been done in response to receiving an instruction for rotating the guide for a next irradiation section of the plurality of irradiation sections before irradiation with the treatment laser light to all the plurality of irradiation spots in the currently-treated section is completed.

11. The ophthalmic laser treatment device according to claim 9 or 10, wherein
the control unit is further configured to move, by controlling the irradiation position moving unit, the irradiation position of the treatment laser light and the aiming light in a direction opposite to the forward direction defined by the irradiation plan each time the control unit rotates the guide for a next irradiation section of the plurality of irradiation sections.

12. An ophthalmic laser treatment device that irradiates a tissue of a patient's eye with treatment laser light in response to receiving an irradiation execution instruction, comprising:
a treatment laser light source that is configured to emit treatment laser light;
an aiming light source that is configured to emit aiming light for an operator to recognize a scheduled irradiation position for the treatment laser light on the tissue;
an irradiation position moving unit that is configured to move, on the tissue, an irradiation position at which the treatment laser light and the aiming light are emitted;
an observation optical system that is configured to present an observation image of the tissue to the operator;
a display unit that is configured to display an image by superimposing the image on the observation image observed by the operator; and
a control unit, wherein
the control unit is configured to execute:
an irradiation plan acquisition step of acquiring an irradiation plan that defines an irradiation order of irradiations with the treatment laser light to a plurality of irradiation spots at which the treatment laser light is scheduled to be emitted when irradiating the patient's eye with the treatment laser light using a contact lens having a reflective surface that reflects the treatment laser light and the aiming light in a direction intersecting an optical axis;
a guide display step of controlling the display unit to display a guide in accordance with a progress of the irradiation plan, wherein the guide is used to align a target position on the tissue with a next irradiation spot of the plurality of irradiation spots at which the treatment laser light is scheduled to be emitted next time, and
at the guide display step, the control unit is further configured to execute:
when irradiation with the treatment laser light to n irradiation spots of the plurality of irradiation spots is completed, a non-adjacent position change step of changing a next aiming point, which is used to set the target position on the tissue, to a non-adjacent position, wherein the non-adjacent position is different from an adjacent position that is offset from a previous irradiation spot of the plurality of irradiation spots by n intervals of the plurality of irradiation spots in a forward direction defined by the irradiation plan; and
when executing the non-adjacent position change step, a target position indicator display step of controlling the display unit to display a target position indicator, wherein the target position indicator indicates the tissue at the adjacent position as the target position with which the next aiming point of the guide is aligned.

13. The ophthalmic laser treatment device according to claim 12, wherein
the control unit is further configured to control the display unit, at the target position indicator display step, to display the target position indicator as a mark that surrounds the target position, which is the adjacent position, on the tissue.

14. The ophthalmic laser treatment device according to claim 12 or 13, wherein
the control unit is further configured to control the display unit to remove the displayed target position indicator upon receiving the irradiation execution instruction after the target position indicator was displayed on the display unit at the target position indicator display step.

15. The ophthalmic laser treatment device according to any one of claims 12 to 14, further comprising
an imaging unit that is configured to capture the observation image on which an image is superimposed by the display unit, and
the control unit is further configured to control the display unit to display, to the operator, a reference image that is captured by the imaging unit when displaying the target position indicator at the target position indicator display step.

16. The ophthalmic laser treatment device according to any one of claims 12 to 15, wherein
the control unit is further configured to cause a position at which the target position indicator is superimposed on the observation image on the display unit to follow the target position on the observation image in accordance with movement of the observation image.

17. The ophthalmic laser treatment device according to any one of claims 12 to 16, wherein
the control unit is further configured to move the irradiation position of the treatment laser light and the aiming light to an adjacent position that is offset by n intervals of the plurality of irradiation spots in a forward direction defined by the irradiation plan each time irradiation with the treatment laser light to n irradiation spots of the plurality of irradiation spots is completed.

18. The ophthalmic laser treatment device according to any one of claims 12 to 17, wherein
the control unit is further configured to:
control the display unit to display a next aiming indicator indicating the next aiming point in the guide to set the target position on the tissue at which the treatment laser light is scheduled to be emitted next time; and
move the next aiming indicator to an adjacent position that is offset by n intervals of the plurality of irradiation spots in a forward direction defined by the irradiation plan each time irradiation with the treatment laser light to n irradiation spots of the plurality of irradiation spots is completed.

19. The ophthalmic laser treatment device according to any one of claims 12 to 18, wherein
the tissue of the patient's eye has an arc-shape or an annular shape,
the tissue is divided into a plurality of irradiation sections each having an arc shape,
irradiations with the treatment laser light a predetermined number M (M>=2) of times are scheduled to be performed to each of the plurality of irradiation sections while a displayed position and an angle of the guide on the display unit are fixed, and
each time irradiation with the treatment laser light to all the plurality of irradiation spots in a currently-treated section of the plurality of irradiation sections is completed, the control unit is further configured to:
execute the non-adjacent position change step by rotating the entire guide for a next irradiation section of the plurality of irradiation sections by an angle corresponding to one irradiation section in a forward direction defined by the irradiation plan; and
execute the target position indicator display step.

20. The ophthalmic laser treatment device according to claim 19, wherein
the control unit is further configured to move, by controlling the irradiation position moving unit, the irradiation position of the treatment laser light and the aiming light in a direction opposite to the forward direction defined by the irradiation plan each time the control unit rotates the guide for a next irradiation section of the plurality of irradiation sections.
